# EUROPÄISCHE PATENTSCHRIFT

(11) **EP 0 579 124 B1**
(45) Veröffentlichungstag und Bekanntmachung des Hinweises auf die Patenterteilung: **16.10.1996**
(21) Anmeldenummer: 93110979.7
(22) Anmeldetag: 09.07.1993
(51) Int. Cl.: C07C 251/48, A01N 37/50

(54) **Substituierte Oximether, Verfahren zu ihrer Herstellung und ihre Verwendung zur Bekämpfung von Schädlingen und Pilzen**
Substituted oxime-ethers, process for their production and their use as pesticides
Ethers d'oximes substitués, procédé pour leur préparation et leur application comme pesticides

(30) Priorität: 15.07.1992 DE 4223210; 30.09.1992 DE 4232816; 31.03.1993 DE 4310495
(43) Veröffentlichungstag der Anmeldung: 19.01.1994
(60) Teilanmeldung: 95108494.6
(73) Patentinhaber: BASF Aktiengesellschaft, 67063 Ludwigshafen (DE)
(72) Erfinder: Oberdorf, Klaus, Dr., D-6900 Heidelberg (DE); Sauter, Hubert, Dr., D-6800 Mannheim 1 (DE); Grammenos, Wassilios, Dr., D-6700 Ludwigshafen (DE); Kirstgen, Reinhard, Dr., D-6730 Neustadt (DE); Harries, Volker, Dr., D-6710 Frankenthal (DE); Lorenz, Gisela, Dr., D-6730 Neustadt (DE); Ammermann, Eberhard, Dr., D-6148 Heppenheim (DE); Gold, Randall Evan, Dr., D-6706 Wachenheim (DE); Siegel, Wolfgang, Dr., D-6800 Mannheim (DE); Harreus, Albrecht, Dr., D-6700 Ludwigshafen (DE)

(56) Entgegenhaltungen:
- EP-A- 0 253 213
- EP-A- 0 386 561
- EP-A- 0 398 692
- EP-A- 0 463 488

## Beschreibung

Die vorliegende Erfindung betrifft neue substituierte Oximether der allgemeinen Formel l, 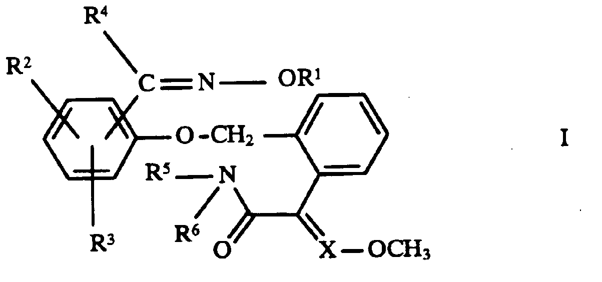 in der
R¹
C₁-C₆-Alkyl, C₃-C₆-Alkenyl, C₃-C₄-Alkinyl, C₁-C₆-Halogenalkyl, C₃-C₆-Halogenalkenyl, C₁-C₄-Alkoxy-C₁-C₆-alkyl, C₃-C₆-Cycloalkyl, C₃-C₆-Cycloalkyl-C₁-C₄-alkyl, Cyan-C₁-C₆-alky, C₁-C₆-Alkoxycarbonyl-C₁-C₆-alkyl, Aryl-C₁-C₆-alkyl, Heteroaryl-C₁-C₆-alkyl, Aryl-C₃ C₆-alkenyl oder Aryloxy-C₁-C₆-alkyl bedeutet, wobei der aromatische oder heteroaromatische Ring gegebenenfalls durch einen oder mehrere der folgenden Reste substituiert ist: C₁-C₄-Alkyl, C₁-C₂-Halogenalkyl, C₃-C₆-Cycloalkyl, C₁-C₄-Alkoxy, C₁-C₂-Halogenalkoxy, Halogen, Aryl, Aryloxy, R² und R3
gleich oder verschiedenen sind und Wasserstoff, C₁-C₄-Alkyl, C₁-C₂-Halogenalkyl, C₁-C₄-Alkoxy, C₁-C₂-Halogenalk- oxy, Halogen, Cyano oder Nitro bedeuten,
R4
Wasserstoff, C₁-C₆-Alkyl, C₁-C₆-Cycloalkyl, C₁-C₇-Halogenalkyl oder Aryl bedeutet, wobei der aromatische Ring gegebenenfalls durch einen oder mehrere der folgenden Reste substituiert ist:
C₁-C₄-Alkyl, C₁-C₂-Halogenalkyl, C₁-C₄-Alkoxy, C₁-C₂-Halogenalkoxy, Halogen, Cyano oder Nitro,
R⁵ und R6
gleich oder verschieden sind und Wasserstoff oder C₁-C₄-Alkyl bedeuten, und
X CH oder N bedeutet.

Außerdem betrifft die Erfindung Verfahren und Zwischenprodukte zur Herstellung der Verbindungen I, sie enthaltende Mittel zur Bekämpfung von Schadpilzen und deren Verwendung, sie enthaltende Mittel zur Bekämpfung von Schädlingen sowie die Verwendung von Verbindungen der allgemeinen Formel l, zur Bekämpfung von Schädlingen.

Es ist bekannt, Oximether wie zum Beispiel das 2-(2'-Methylphenoxymethyl) -phenyl-glyoxylsäuremethylester-O-methyl oder das 2-(2'-Methyl-4'-(methoximinoeth-1 "-yl)-phenoxymethyl) -phenylglyoxylsäuremethylester-O-methyloxim als Fungizide zu verwenden (EP-A 253 213; EP-A 398 692).

Des weiteren sind aus der EP-A 386 561 der Formel l ähnliche Verbindungen, in denen an Stelle der R⁵R⁶N-Gruppe die Methoxygruppe steht, als fungizide Wirkstoffe bekannt

Aufgabe der vorliegenden Erfindung waren neue Verbindungen mit verbesserter und breiterer Anwendbarkeit im Pflanzenschutz.

Demgemäß wurden die eingangs definierten Verbindungen I, Verfahren und Zwischenprodukte zu ihrer Herstellung sowie sie enthaltende Mittel und deren Verwendung zur Bekämpfung von Schadpilzen sowie Mittel zur Bekämpfung von Schädlingen gefunden.

Die in der allgemeinen Formel l aufgeführten Reste können beispielsweise folgende Bedeutung haben:
R¹
kann z B. C₁-C₆-Alkyl (C₁-C₄-Alkyl) (z B. Methyl, Ethyl, n- oder iso-Propyl, n-, iso-, sec-oder tert. -Butyl, n-, iso-, sec.-, tert.-oder neo-Pentyl, Hexyl), C₃-C₆-Alkenyl (z.B. Allyl, 2-Butenyl, 3-Butenyl, 1-Methyl-2-propenyl, 2-Methyl-2-propenyl), C₃-C₄-Alkinyl (z.B. Propargyl, 2-Butinyl), C₇-C₆-Halogenalkyl, (z.B. 2-Fluorethyl), C₃-C₆-Halogenalkenyl (z.B. 3-Chlorallyl), C₁-C₄-Alkoxy-C₁-C₆-alkyl (z.B. 2-Methoxyethyl, 3-Ethoxypropyl), C₃-C₆-Cycloalkyl (z B. Cyclopropyl, Cyclobutyl, Cyclopentyl, Cyclohexyl), C₃-C₆-Cycloalkyl-C₁-C₄-alkyl (z.B. Cyclopropylmethyl, Cyclohexylmethyl), Cyan-C₁-C₆-alkyl (z.B. Cyanmethyl, 3-Cyanpropyl), C₁-C₆-Alkoxycarbonyl-C₁-C₆-alkyl (z.B. Ethoxycarbonylmethyl, tert.-Butoxycarbonylmethyl, tert.-Butoxycarbonylpropyl), Aryl(Phenyl)-C₁-C₆-alkyl (z.B. Benzyl, 2-Phenylethyl, 3-Phenylpropyl, 4-Phenylbutyl), Heteroaryl-(Pyridyl, Thienyl)-C₁-C₆-alkyl (z.B. Pyrid-3-yl-methyl, Thien-2-yl-methyl), Aryl-(Phenyl)-C₃-C₆-alkenyl (z.B. 4-Phenyl-2-butenyl, 4-Phenyl-3-butenyl), Aryloxy(Phenoxy)-C₁-C₆-alkyl (z.B. Phenoxymethyl, Phenoxyethyl, Phenoxypropyl, Phenoxybutyl, Naphthoxymethyl, Naphthoxyethyl) sein,

wobei der aromatische (Phenyl) oder heteroaromatische (Pyridyl, Thienyl) Ring gegebenenfalls durch einen oder mehrere z. B. 1 bis 5, insbesondere 1 bis 3 der folgenden Reste substituiert ist:
C₁-C₄₋Alkyl (z.B. Methyl, Ethyl, Propyl, Butyl), C₁-C₂-Halogenalkyl, (z.B. Trifluormethyl, Trichlormethyl), C₃-C₆-Cycloalkyl (z.B. Cyclopropyl, Cyclobutyl, Cyclopentyl, Cyclohexyl), C₁-C₄-Alkoxy (z. B. Methoxy, Ethoxy, Propoxy, Butoxy), C₁-C₂-Halogenalkoxy (z. B. Trifluormethoxy), Halogen (z.B. Fluor, Chlor, Brom), Aryl (z.B. Phenyl), Aryloxy (z.B. Phenoxy),
R² und R3
können gleich oder verschieden sein und Wasserstoff, Cᵢ-C₄-Alkyl (z.B. Methyl, Ethyl, n- oder iso-Propyl, Butyl), C₁-C₂-Halogenalkyl, (z.B. Trifluormethyl, Trichlormethyl), C₁-C₄-Alkoxy (z.B. Methoxy, Ethoxy, n-oder iso-Propoxy, Butoxy), C₁-C₂-Halogenalkoxy (z. B. Trifluormethoxy), Halogen (z.B. Fluor, Chlor, Brom, Jod), Cyano oder Nitro sein, _{R}4
kann z. B. C₁-C₆-Alkyl, (C₁-C₄-Alkyl) (z.B. Methyl, Ethyl, n- oder iso-Propyl, n-, iso-, sec -oder tert.-Butyl, n-, iso, sec.- tert oder neo-Pentyl, Hexyl), C₁-C₇-Halogenalkyl (z.B Trifluormethyl, Trichlormethyl, Chlormethyl, 2-Chlorethyl, 3-Chlorpropyl, 3-Brompropyl, 4-Chlorbutyl, 4-Brombutyl, 5-Chlorpentyl, 5-Brompentyl, 6-Chlorhexyl, 6-Bromhexyl), C₃-C₆-Cycloalkyl (z.B. Cyclopropyl, Cyclobutyl, Cyclopentyl und Cyclohexyl) sein oder Aryl (z.B. Phenyl) sein, wobei der aromatische Ring gegebenenfalls durch einen oder mehrere z.B 1 bis 5, insbesondere 1 bis 3 der folgenden Reste substituiert ist: C₁-C₄-Alkyl (z.B. Methyl, Ethyl, Propyl, Butyl), C₁-C₂-Halogenalkyl (z.B. Trifluormethyl, Trichlormethyl), C₁-C₄-Alkoxy (z.B. Methoxy, Ethoxy, Propoxy, Butoxy), C₁-C₂-Halogenalkoxy (z.B. Difluormethoxy, Trifluormethoxy), Halogen (z.B. Fluor, Chlor, Brom, Jod) Cyano oder Nitro.
R⁵ und R6
können gleich oder verschieden sein und Wasserstoff oder C₁-C₄-Alkyl (z. B. Methyl, Ethyl, n- oder iso-Propyl, Butyl) sein. Bevorzugt sind Verbindungen mit R⁵ = Wasserstoff und R⁶ = Methyl,
X kann CH oder N bedeuten und

Der Rest -C(R⁴)=N-O-R¹ kann am Phenylrest im Hinblick auf den Rest -O-CH₂- in 2-, oder in 3- oder bevorzugt in 4-Stellung stehen.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographie, in die einzelnen Komponenten getrennt werden. Sowohl die einzelnen isomeren Verbindungen als auch ihre Gemische werden von der Erfindung umfaßt und sind als Fungizide und Schädlingsbekämpfungsmittel brauchbar. Bezüglich der Gruppierung -C(CONR⁵R⁶) = X-OCH₃ sind diejenigen Verbindungen bevorzugt, in denen die Gruppen CONR⁵R⁶ und OCH3 an der C=X-Doppelbindung E-Konfiguration besitzen. Bezüglich der Gruppierung -C(R⁴)=N-OR¹ sind diejenigen Verbindungen bevorzugt, in denen R⁴ und OR¹ an der C=N-Doppelbindung cis-ständig sind und in denen deshalb bei kleinen Substituenten wie z.B. Methyl die C=N-Doppelbindung E-Konfiguration hat.

Die Darstellung der neuen Verbindungen der Formel I erfolgt beispielsweise so, daß man einen substituierten Oximether der allgemeinen Formel ll, wobei L = C₁-C₄-Alkoxy, Hydroxy oder Halogen wie Chlor oder Brom bedeutet, mit einem primären oder sekundären Amin der Formel HNR⁵R⁶ umsetzt.

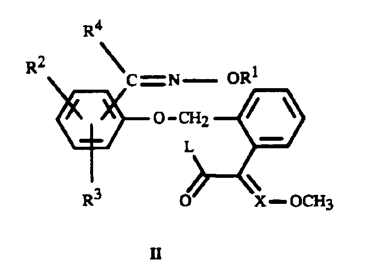

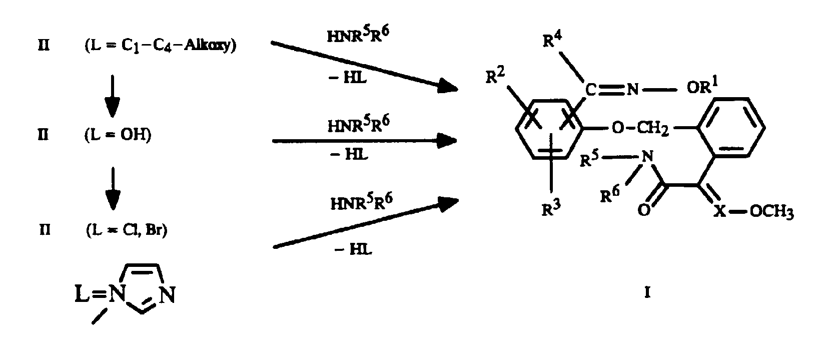

Verbindungen der Formel ll, wobei L = C₁-C₄-Alkoxy bedeutet, sind aus EP 386561 bekannt oder können analog zu dort beschriebenen Verfahren hergestellt werden. Daraus lassen sich leicht die entsprechenden Carbonsäuren ll (L OH) nach gängigen Verfahren (s. z.B. Houben Weyl, Bd E 5, S. 223 - 254; Org. Reactions 24, (1976), S. 187 - 224) herstellen. Diese können anschließend in aktivierte Carbonsäurederivate wie etwa die Säureimidazolide ll mit L = Imidazol-1-yl oder die Säurehalogenide ll mit L = CI, Br überführt werden (s. Z.B. Houben Weyl, Bd VIII, S. 463 ff). Aus den Verbindungen der Formel ll resultieren durch Umsetzung mit primären oder sekundären Aminen HNR⁵R⁶ die entsprechenden Amide der allgemeinen Formel l (s. z.B. Houben Weyl, Bd E 5, S 941 - 977, S. 983 - 991; Houben Weyl, Bd VIII, S. 654 ff).

R¹, _{R}², _{R}³, R⁴, R⁵, R⁶ und X haben die oben genannten Bedeutungen.

Die folgenden Beispiele und Vorschriften sollen die Herstellung der neuen Wirkstoffe und ihrer Vorprodukte erläutern.

### Herstellungsbeispiel 1

2-[2'-Methyl-4'-(methoxyiminoeth-1 "-yl)-phenoxymethyl]-phenylglyoxylsäure-methylamid-O-methyloxim
a) 225,3 g (1,5 mol) 4-Hydroxy-3-methyl-acetophenon werden in 600 ml trockenem Methanol gelöst. 150,3 g (1,8 mol) Methoxyaminhydrochlorid und 100 g Molekularsieb werden zugesetzt. Es wird 12 Stunden bei Raumtemperatur (20°C) gerührt. Das Molekularsieb wird abfiltriert. Das Filtrat wird eingeengt. Der verbleibende Rückstand wird in Dichlormethan aufgenommen. Die organische Phase wird mit Wasser gewaschen, getrocknet und eingeengt. Das erhaltene Festprodukt wird mit Pentan gewaschen und anschließend getrocknet. Man erhält 252 g (94 %) 4-Hydroxy-2-methyl-acetophenon-O-methyloxim in Form eines farblosen kristallinen Feststoffs (Fp : 96 - 98°C).
b) 89,6 g (0,5 mol) 4-Hydroxy-3-methyl-acetophenon-O-methyloxim werden unter Stickstoff in 300 ml trockenem Methanol vorgelegt 90 g (0,5 mol) einer 30 % (Gew.-%) Natriummethanolat-Lösung werden zugetropft. Nach 2 Stunden wird das Methanol abdestilliert. Der Rückstand wird in 700 ml Dimethylformamid gelöst. 15 g Kaliumjodid werden zugesetzt. Anschließend wird bei Raumtemperatur unter Stickstoff eine Lösung von 151,6 g (0,53 mol) 2-(Brommethyl)-phenylglyoxylsäuremethylester-O-methyloxim in 300 ml Methanol zugetropft. Nach etwa 10 Stunden Rühren bei Raumtemperatur wird auf etwa 10 °C abgekühlt und es wird Wasser zugetropft. Der entstandene Niederschlag wird abfiltriert, mit Wasser und Pentan nachgewaschen und getrocknet. Man erhält 153,7 g (80 %) 2-[2'-Methyl-4'-(methoxyiminoeth-1 "-yl)-phenoxymethyl]-phenylglyoxylsäuremethylester-O-methyloxim als farblosen kristallinen Feststoff (Fp.: 138 - 140°C).
c) 4,8 g (0,012 mol) 2-[2'-Methyl-4'-(methoxyiminoeth-1 "-yl)-phenoxymethyl]-phenylglyoxylsäuremethylester-O-methyloxim werden in 32 ml Tetrahydrofuran gelöst und mit 3,6 g (0,047 mol) einer 40 proz. wässrigen Methylaminlösung versetzt. Das Reaktionsgemisch wird anschließend für 6 Stunden bei 40 °C gerührt. Anschließend wird eingeengt. Der Rückstand wird in Methyl-tert.-butylether aufgenommen. Die organische Phase wird mit Wasser gewaschen, getrocknet und erneut eingeengt. Das verbleibende Rohprodukt wird über eine Kieselgel-Säule (Cyclohexan/ Essigsaureethylester = 1/1) chromatographisch gereinigt. Man erhält 3,2 g (67 %) 2-[2'-Methyl-4'-(methoxyiminoeth-1 "-yl)-phenoxymethyl]-phenylglyoxylsäure-methylamid-O-methyloxim in Form farbloser Kristalle (Fp.: 104-105°C, Verbindung 1.007).

### Herstellungsbeispiel 2

### α-[2-(2"-Methyl-4"-(methoxyiminoeth-1 "-yl)-phenoxymethyl)phenyl]-β-methoxy-acrylsäure-methylamid

a) α-(2-Brommethylphenyl)-β-methoxy-acrylsäuremethylester und 4-Hydroxy-3-methyl-acetophenon-O-methylo- xim werden analog Vorschrift b) (Beispiel 1) zu α-[2-(2'-Methyl-4'-(methoxyiminoeth-1 "-yl)-phenoxymethyl)-phenyl)-ß-methoxy-acrylsäure-methylester umgesetzt. Die Verbindung fällt als farbloser Feststoff (Fp.: 118 - 120°C) an.
b) 3 g (0,0078 mol) a-[2-(2'-Methyi-4'-(methoxyiminoeth-1 "-yl)phenoxymethyl)-phenyl]-ß-methoxy-acrylsäure-methylester werden in 15 ml trockenem Pyridin gelöst. Man setzt 5,2 g (0,039 mol) wasserfreies Lithiumjodid zu und rührt für 8 Stunden bei 130 °C. Die Reaktionsmischung wird eingeengt. Der Rückstand wird in Wasser aufgenommen. Die wässrige Phase wird zunächst mit Methyl-tert.-butylether gewaschen und anschließend mit Salzsäure angesäuert. Dann wird die wässrige Phase mit Methyl-tert.-butylether extrahiert. Die Methyl-tert.-butylether-Phase wird mit Wasser gewaschen, über Natriumsulfat getrocknet und eingeengt. Man erhält 2,1 g a-[2-(2'-Methyl-4'-(methoxyiminoeth-1 "-yl)-phenoxymethyl)-phenyl]-ß-methoxyacrylsäure als dunkles Harz, das ohne weitere Reinigung für die Folgereaktionen eingesetzt wird.
c) 2,1 g (0,0056 mol) α-[2-(2'-Methyl-4'-(methoxyiminoeth-1 "-yl)-phenoxymethyl)-phenyl ]-ß-methoxy-acrylsäure und 0,53 g Pyridin werden in 10 ml trockenem Diethylether vorgelegt. Bei 0 - 5°C wird 0,8 g (0,0067 mol) Thionylchlorid zugetropft und für 10 Stunden bei Raumtemperatur gerührt. Es wird filtriert. Das Filtrat wird eingeengt. Man erhält 2 g a-[2-(2'-Methyl-4'-(methoxyiminoeth-1 "-yl)-phenoxymethyl)phenyl]-ß-methoxy-acrylsäurechlorid als dunkles Öl, das ohne weitere Reinigung für die Folgereaktionen eingesetzt wird.
d) 1 g (0,0026 mol) a-[2-(2'-Methyl-4'-(methoxyiminoeth-1 "-yl)phenoxymethyl)-phenyl]-ß-methoxy-acrylsäurechlorid werden in 10 ml Dichlormethan vorgelegt. Man tropft bei 0 - 5 °C eine Lösung aus 1 g (0,032 mol) Methylamin in 10 ml Dichlormethan zu Es wird 10 Stunden bei Raumtemperatur gerührt. Das Reaktionsgemisch wird in 20 ml Dichlormethan aufgenommen, mit Wasser gewaschen, getrocknet und eingeengt. Das verbleibende Rohprodukt wird über eine Kieselgel-Säule (n-Hexan/Aceton = 2/1) chromatographisch gereinigt. Man erhält 0,5 g (50 %) a-[2-(2'-Methyl-4'-(methoxyiminoeth-1 "-yl)-phenoxymethyl)phenyl]-ß-methoxy-acrylsäure-methylamid in Form farbloser Kristalle (Fp.: 96 - 980C, Verbindung 1.006).

In entsprechender Weise lassen sich die in der folgenden Tabelle zusammengestellten Verbindungen I herstellen.

In den daran anschließenden Tabellen 1, 2, 5-11 und 18-22 sind diejenigen Verbindungen 1 zusammengestellt, denen im Hinblick auf ihre biologische Wirksamkeit gegen Schädlinge (pflanzenpatogene Pilze sowie Insekten, Spinnentiere und Nematoden) eine besondere Bedeutung zukommt.

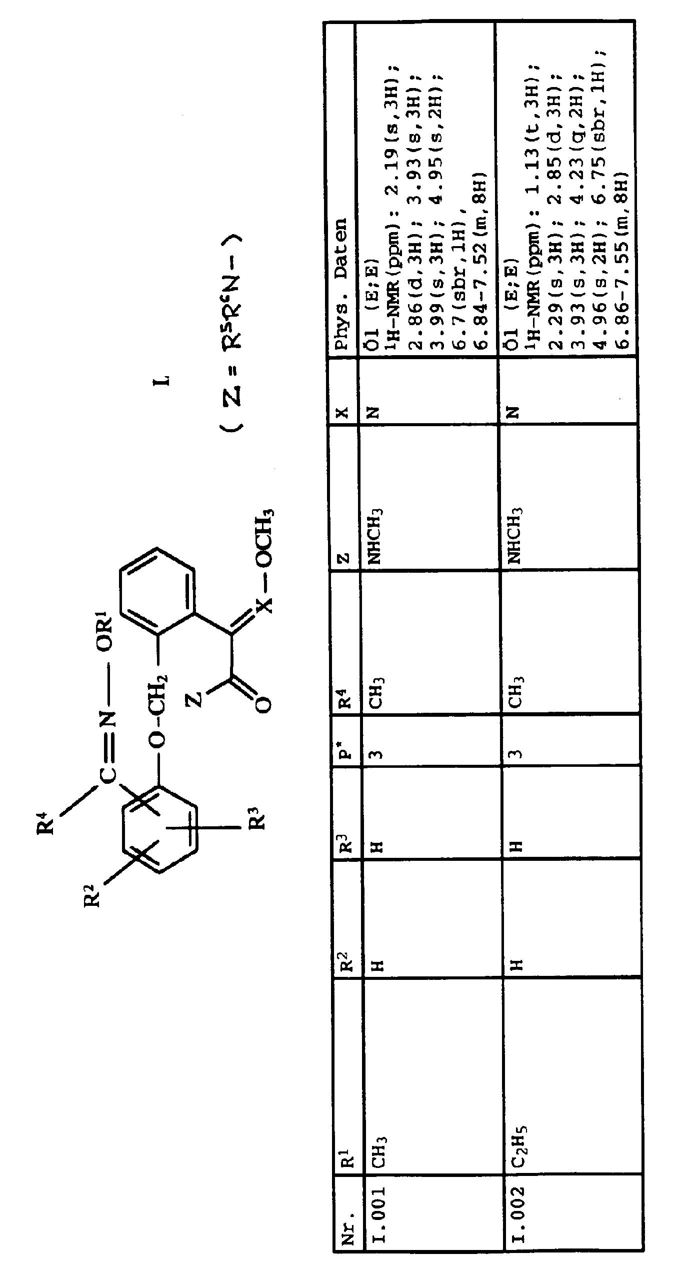

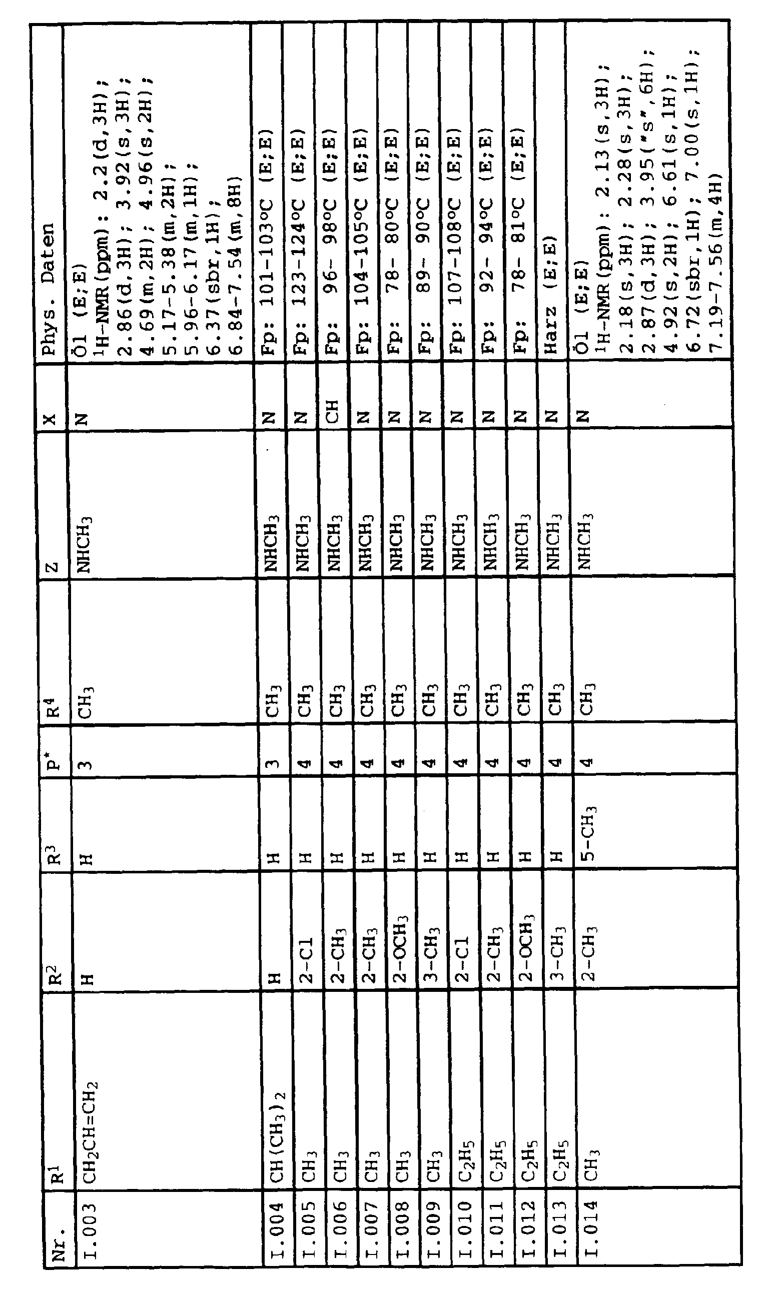

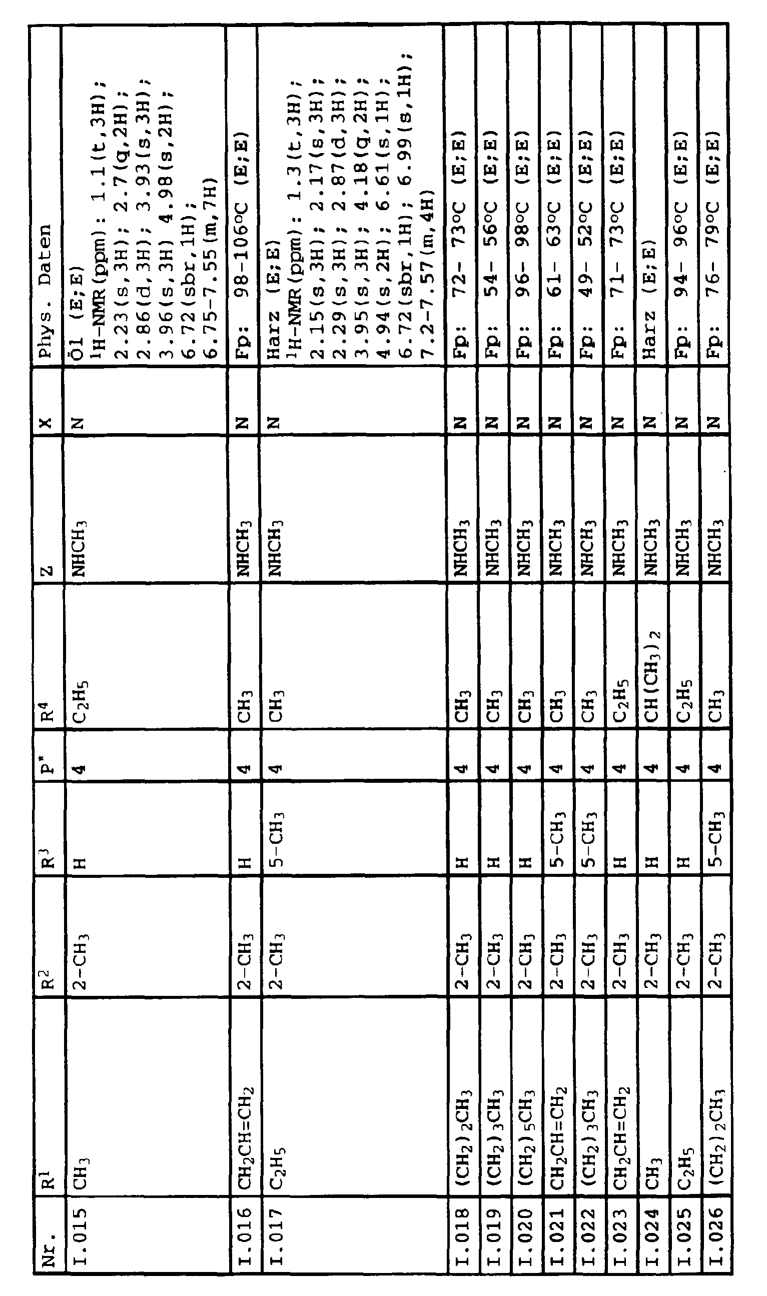

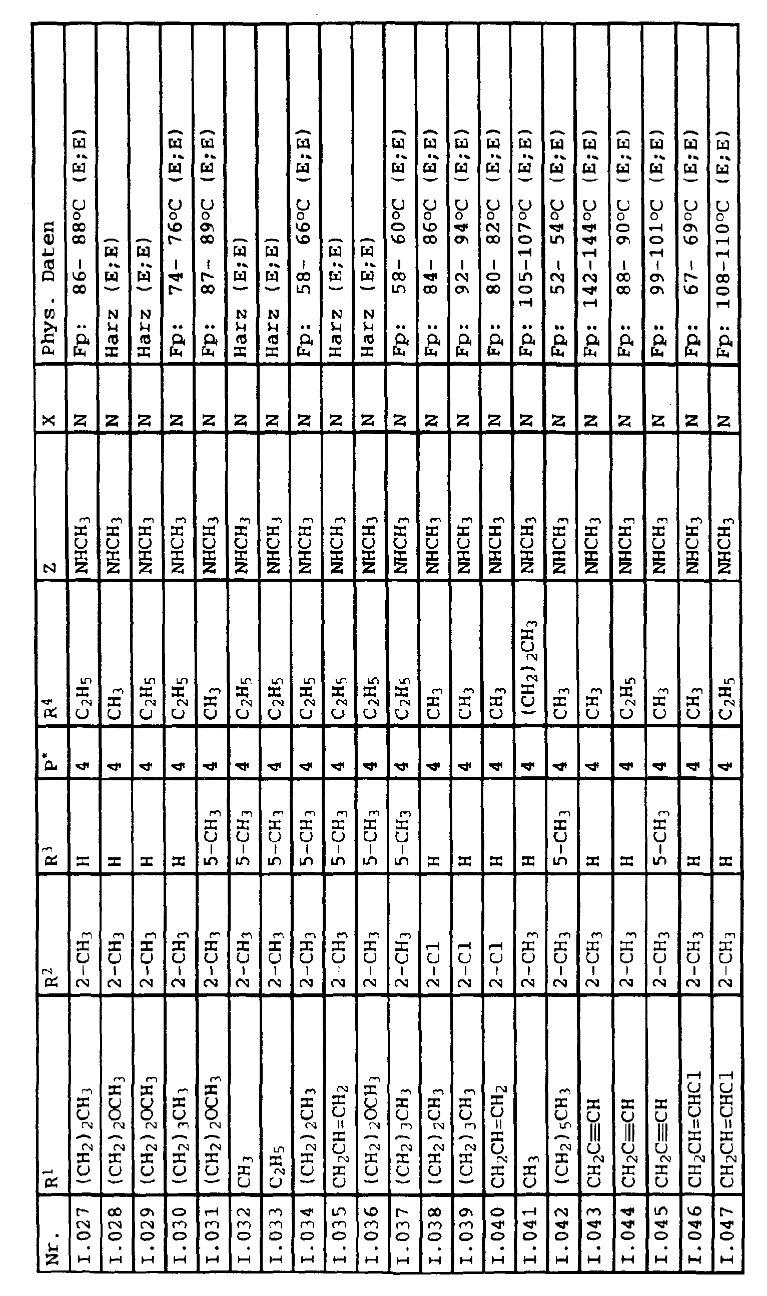

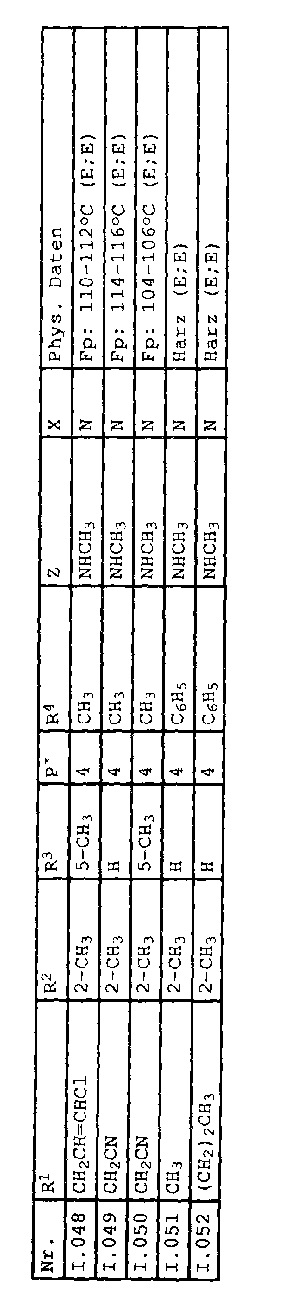

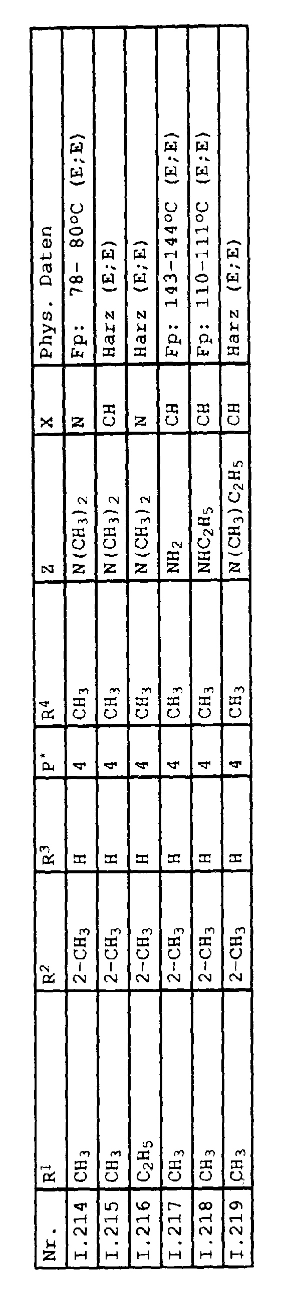

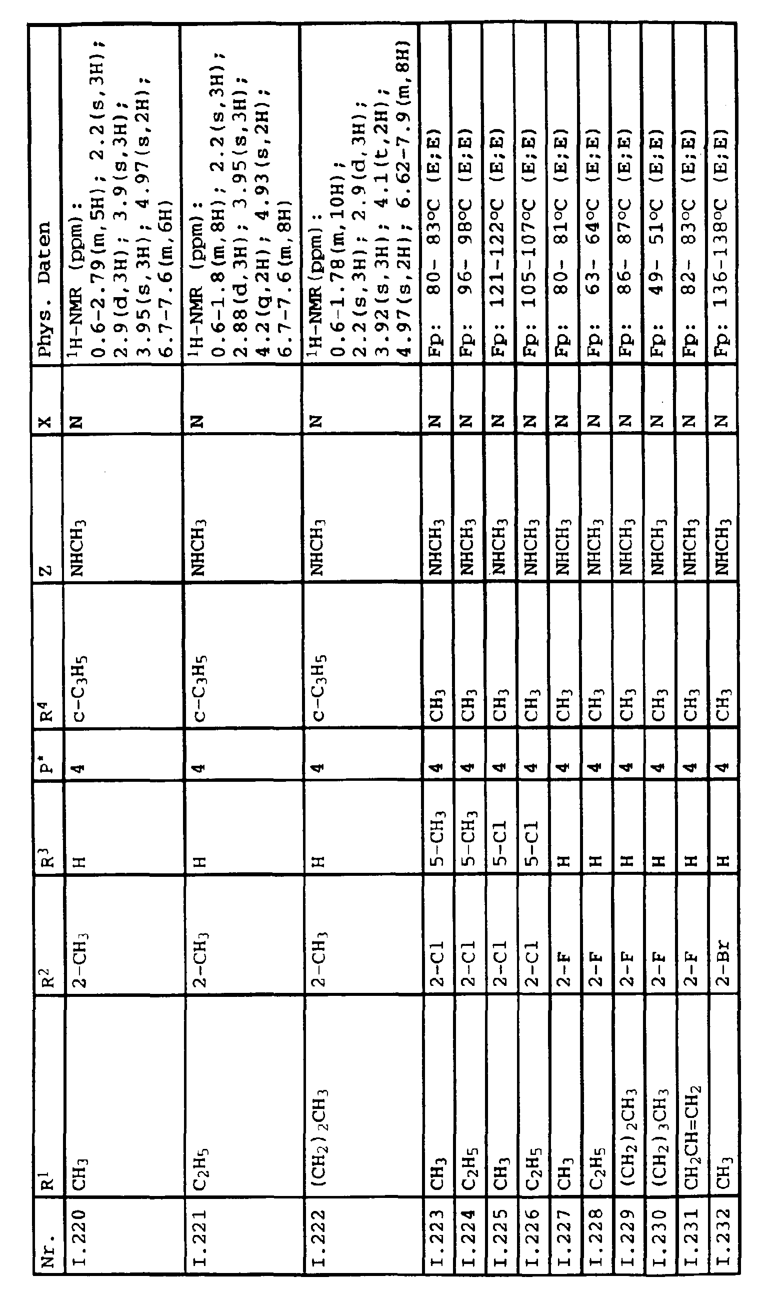

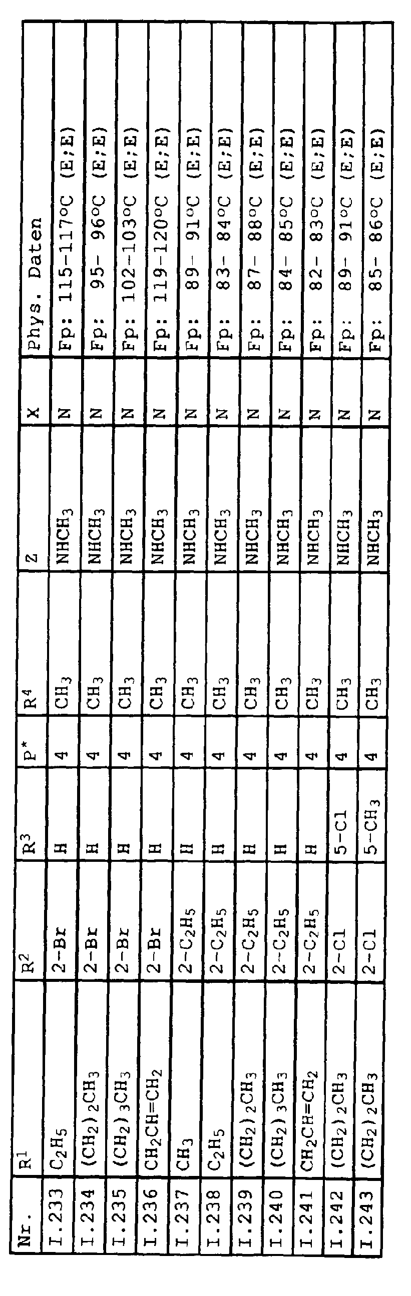

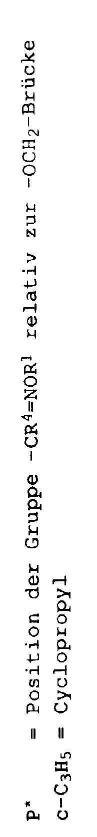

Tabelle 1: Verbindungen der allgemeinen Formel 1.1, in denen die Kombination der Substituenten R¹, R², R³, R⁴ und X für eine Verbindung jeweils einer Zeile der Tabelle A entspricht 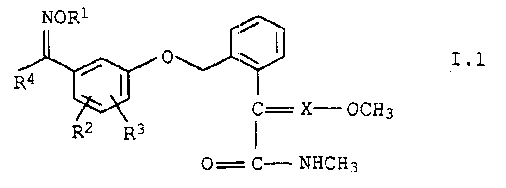
Tabelle 2: Verbindungen der allgemeinen Formel 1.2, in denen die Kombination der Substituenten R¹, R², R³, R⁴ und X für eine Verbindung jeweils einer Zeile der Tabelle B entspricht 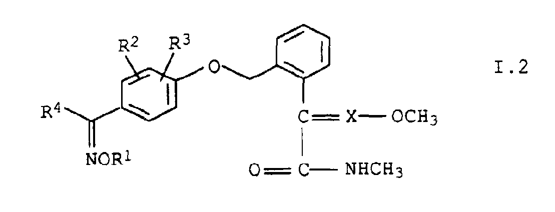
Tabelle 5: Verbindungen der allgemeinen Formel 1.5, in denen die Kombination der Substituenten R¹, R², R³, R⁴, R⁵, R⁶ und X für eine Verbindung jeweils einer Zeile der Tabelle C entspricht 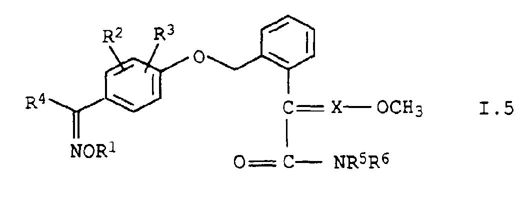
Tabelle 6: Verbindungen der allgemeinen Formel 1.2, in denen R⁴ Cyclopropyl bedeutet und die Kombination der
Substituenten R¹, R², R³ und X für eine Verbindung jeweils einer Zeile der Tabelle D entspricht Tabelle 7: Verbindungen der allgemeinen Formel 1.2, in denen R⁴ Cyclopentyl bedeutet und die Kombination der
Substituenten R¹, R², R³ und X für eine Verbindung jeweils einer Zeile der Tabelle D entspricht Tabelle 8: Verbindungen der allgemeinen Formel 1.2, in denen R⁴ Cyclohexyl bedeutet und die Kombination der
Substituenten R¹, R², R³ und X für eine Verbindung jeweils einer Zeile der Tabelle D entspricht Tabelle 9: Verbindungen der allgemeinen Formel l., in denen R⁴ CF₃ bedeutet und die Kombination der Substituenten R¹, R², R³ und X für eine Verbindung jeweils einer Zeile der Tabelle D entspricht
Tabelle 10: Verbindungen der allgemeinen Formel 1.2, in denen R⁴ CH₂C1 bedeutet und die Kombination der Substituenten R¹, R², R³ und X für eine Verbindung jeweils einer Zeile der Tabelle D entspricht
Tabelle 11: Verbindungen der allgemeinen Formel 1.2, in denen R⁴ CH₂CH₂CI bedeutet und die Kombination der Substituenten R¹, R², R³ und X für eine Verbindung jeweils einer Zeile der Tabelle D entspricht
Tabelle 18: Verbindungen der allgemeinen Formel 1.2, in denen R⁴ für Cyclopropyl und =X- für =N- stehen und die Kombination der Substituenten R¹, R² und R³ für eine Verbindung jeweils einer Zeile der Tabelle E entspricht
Tabelle 19: Verbindungen der allgemeinen Formel 1.2, in denen R⁴ für Cyclopentyl und =X- für =N- stehen und die Kombination der Substituenten R¹, R² und R³ für eine Verbindung jeweils einer Zeile der Tabelle E entspricht
Tabelle 20: Verbindungen der allgemeinen Formel 1.2, in denen R⁴ für Cyclohexyl und =X- für =N- stehen und die Kombination der Substituenten R¹, R² und R³ für eine Verbindung jeweils einer Zeile der Tabelle E entspricht
Tabelle 21: Verbindungen der allgemeinen Formel 1.2, in denen R⁴ für CF₃ und -X- für -N- stehen und die Kombination der Substituenten R¹, R² und R³ für eine Verbindung jeweils einer Zeile der Tabelle E entspricht
Tabelle 22: Verbindungen der allgemeinen Formel 1.2, in denen R⁴ für CH₂CH₂Cl und =X- für =N- stehen und die Kombination der Substituenten R¹, R² und R³ für eine Verbindung jeweils einer Zeile der Tabelle E entspricht

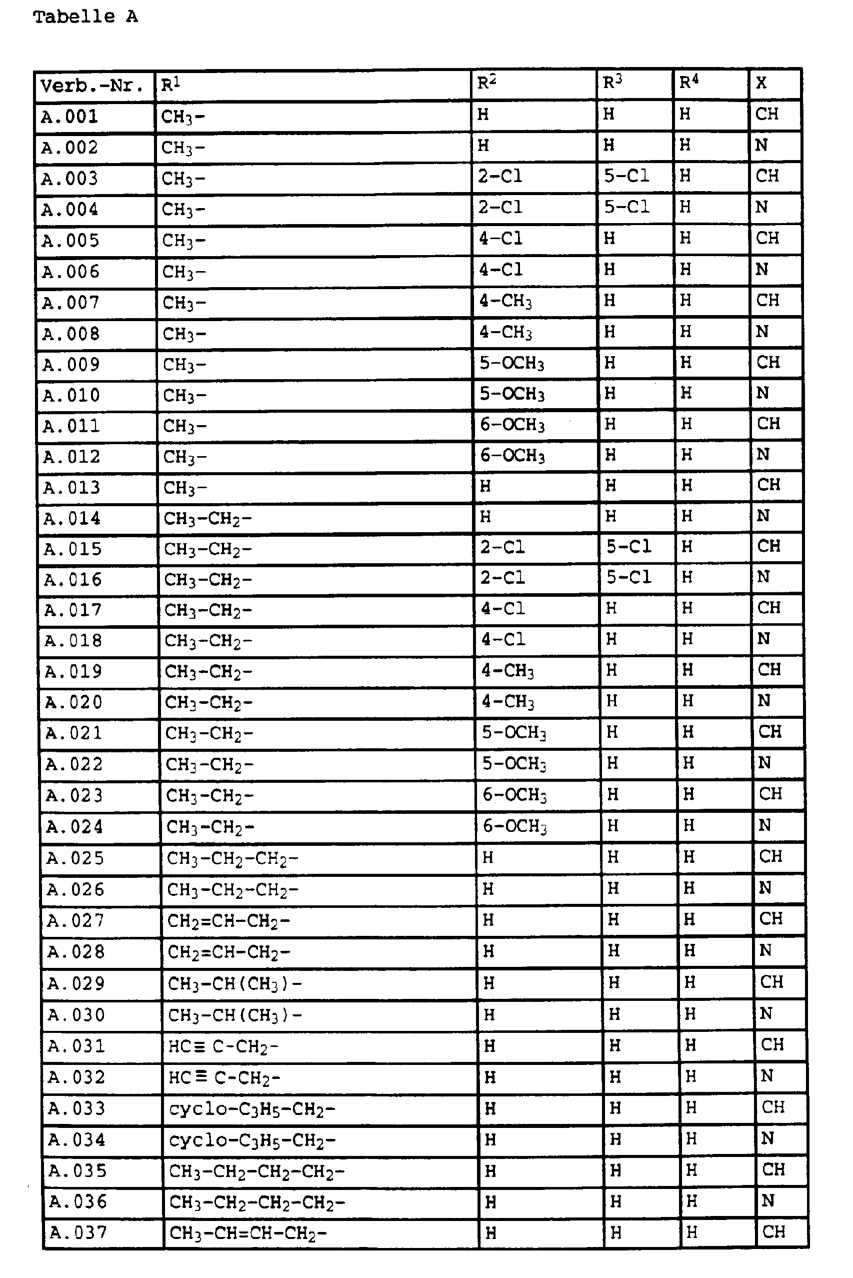

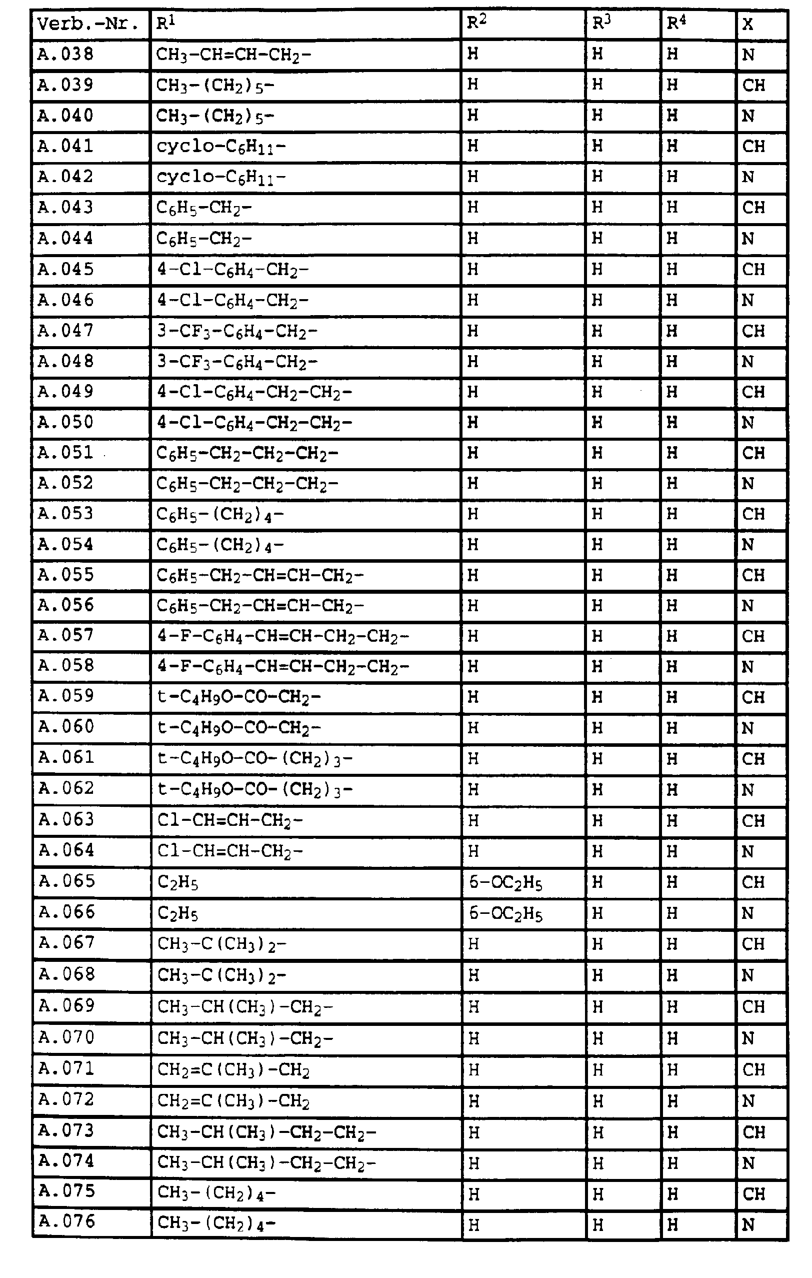

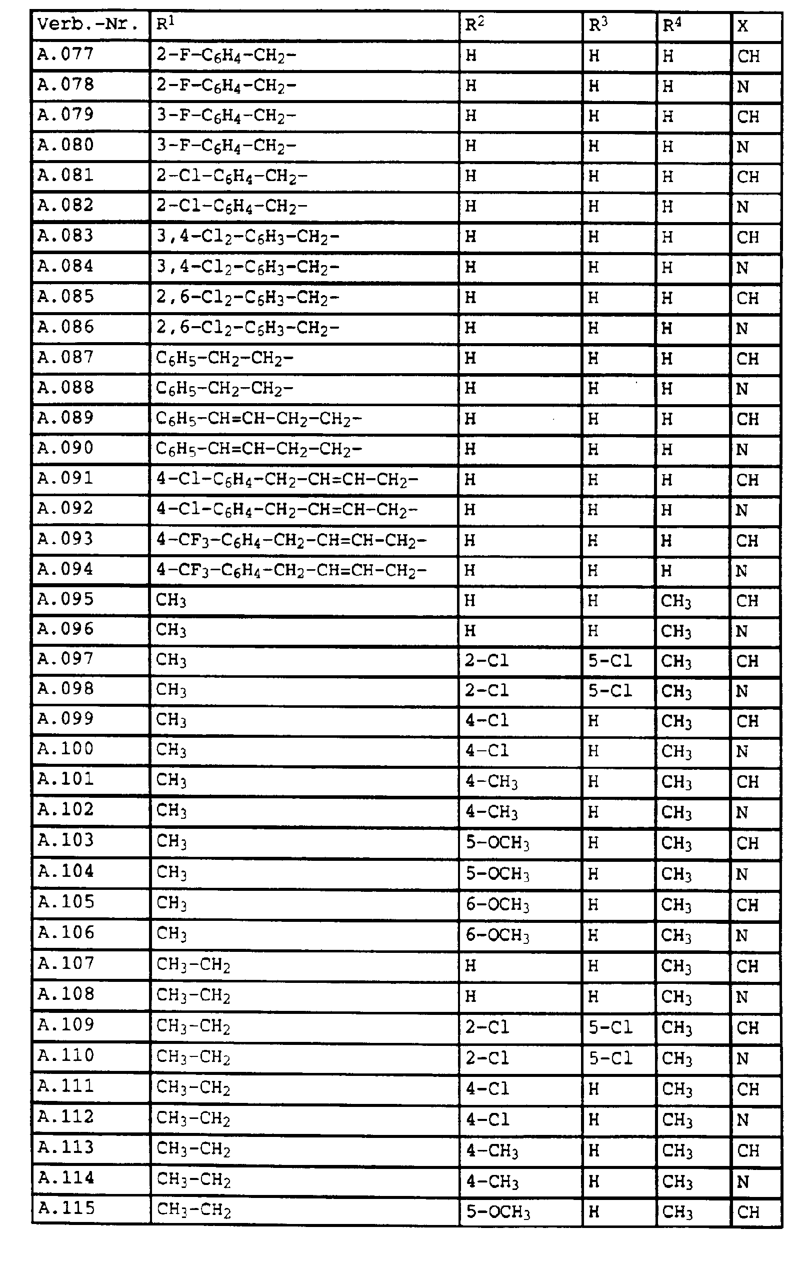

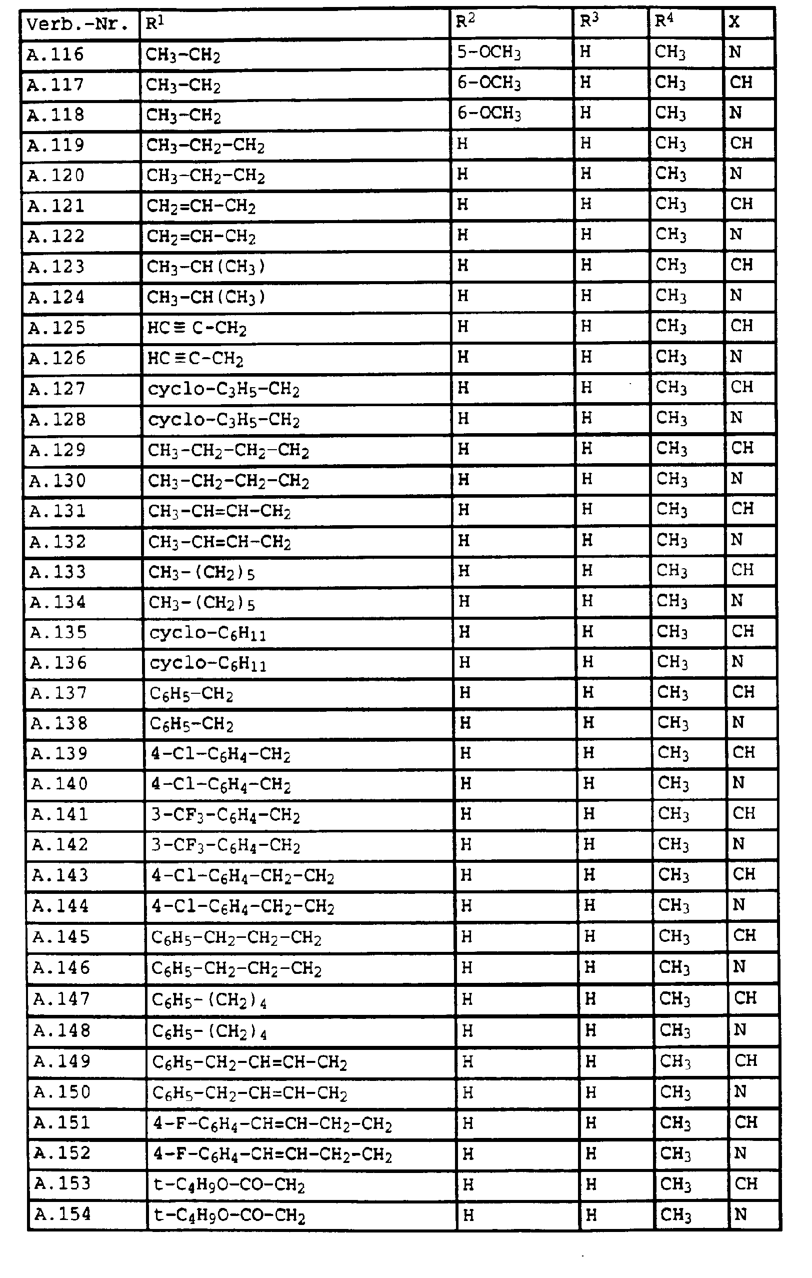

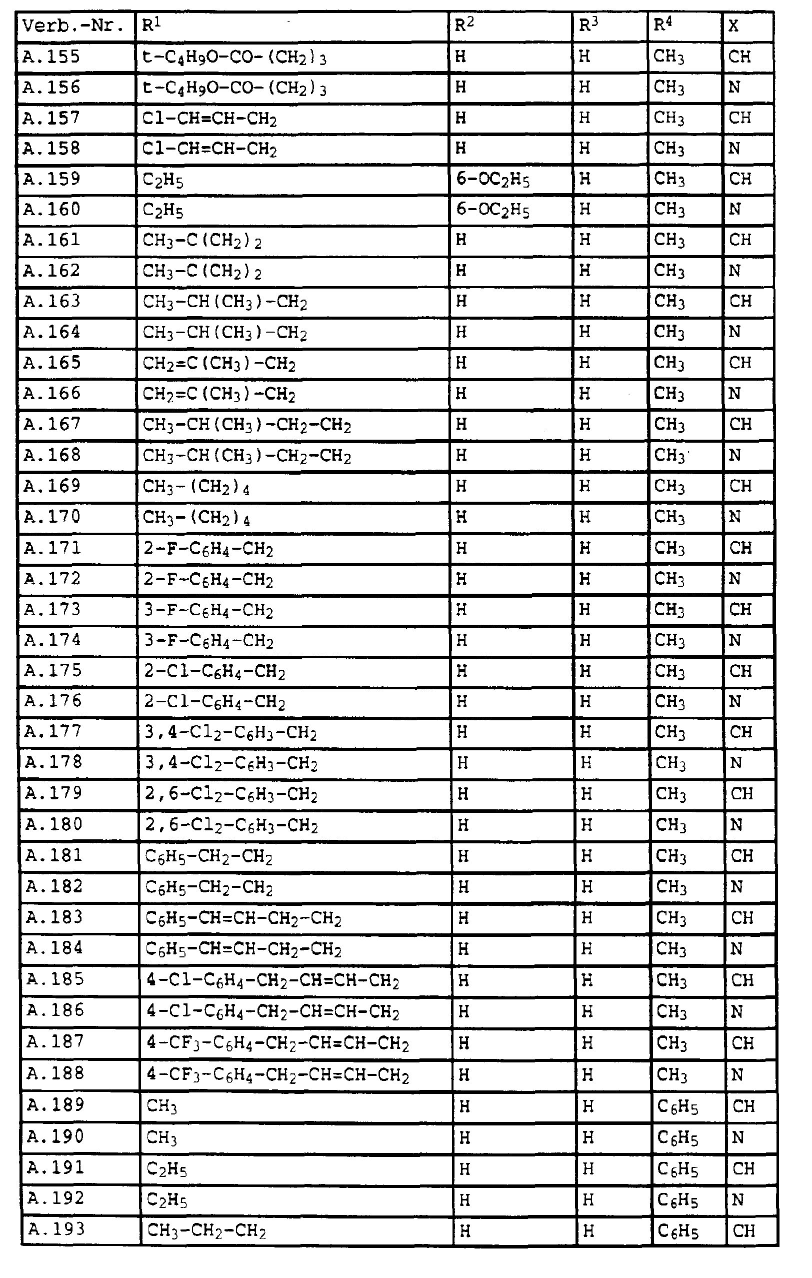

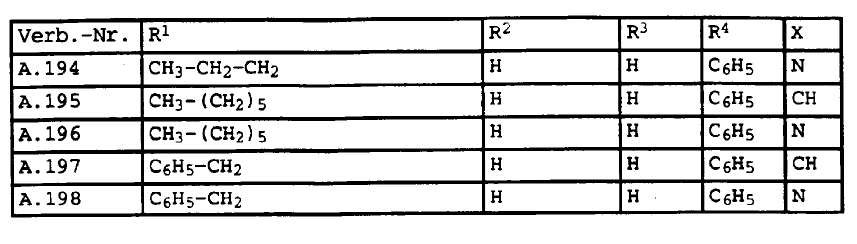

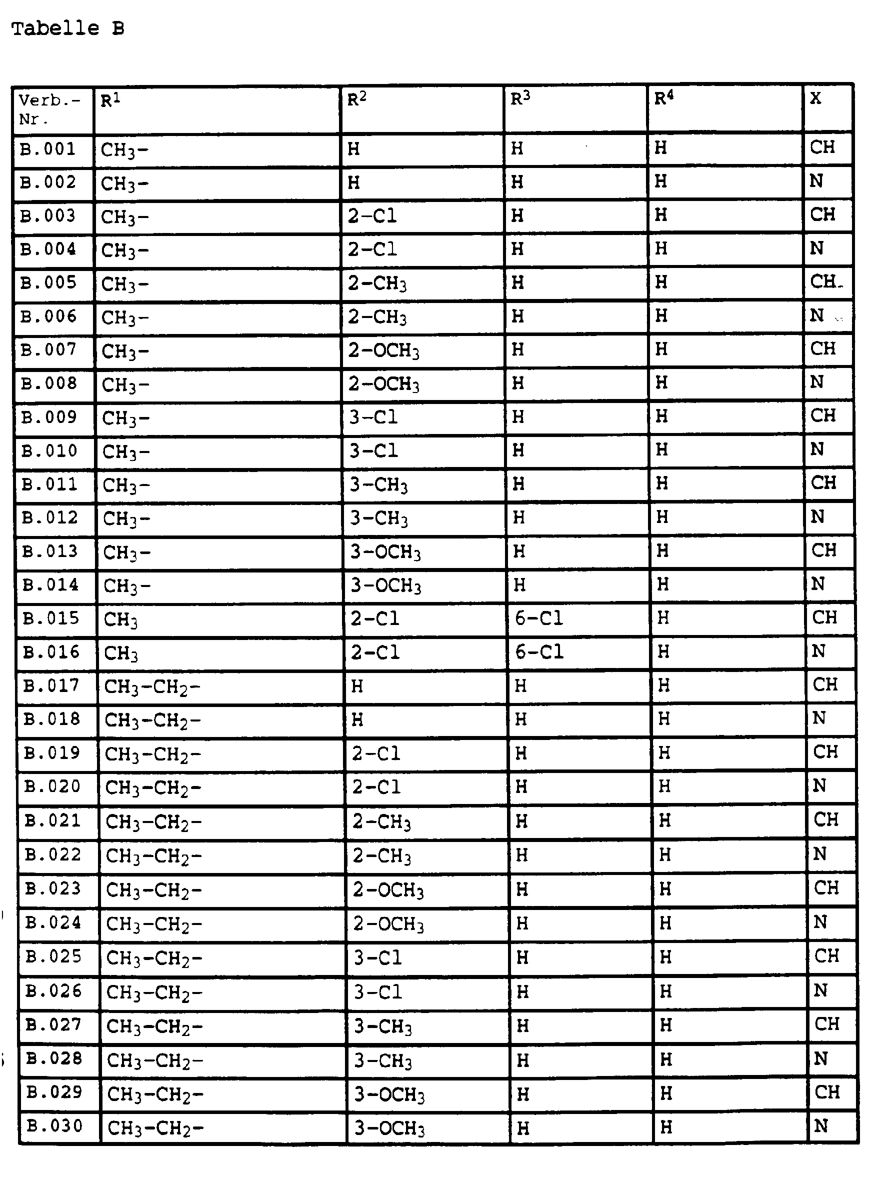

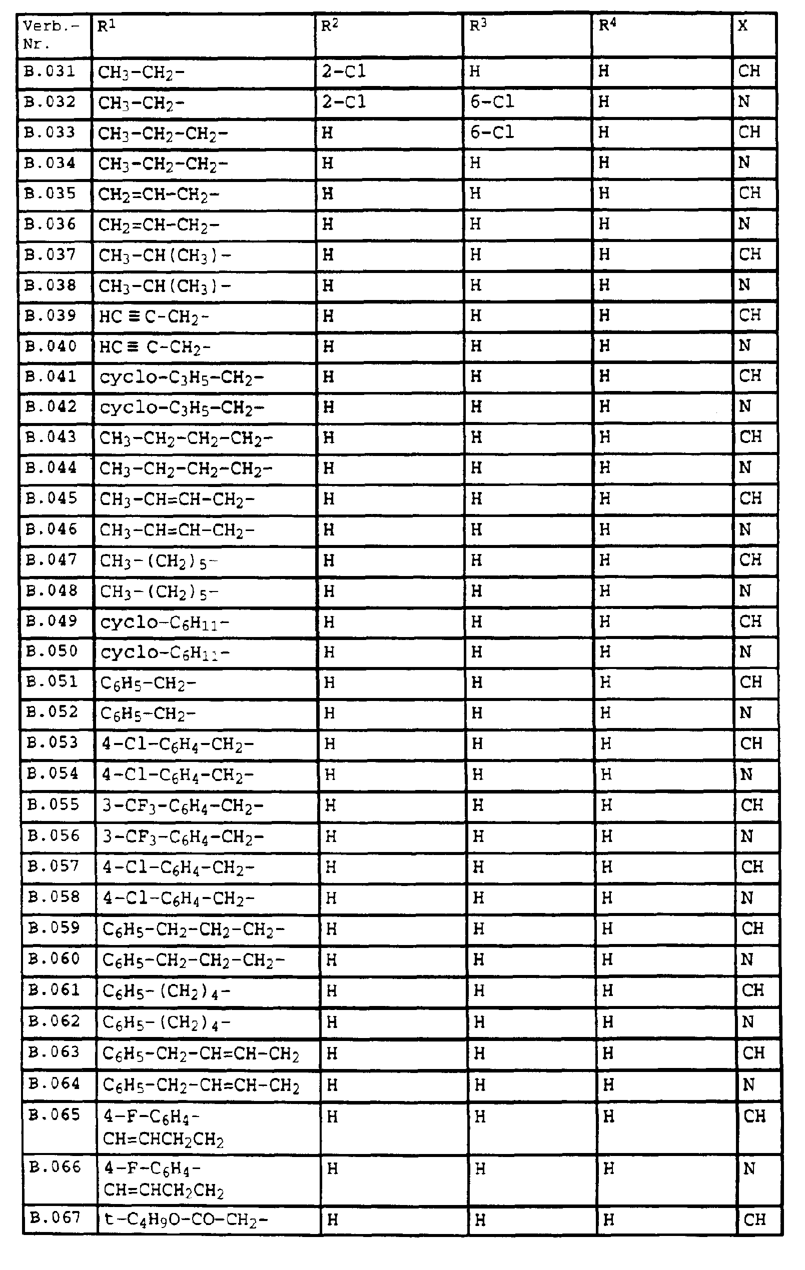

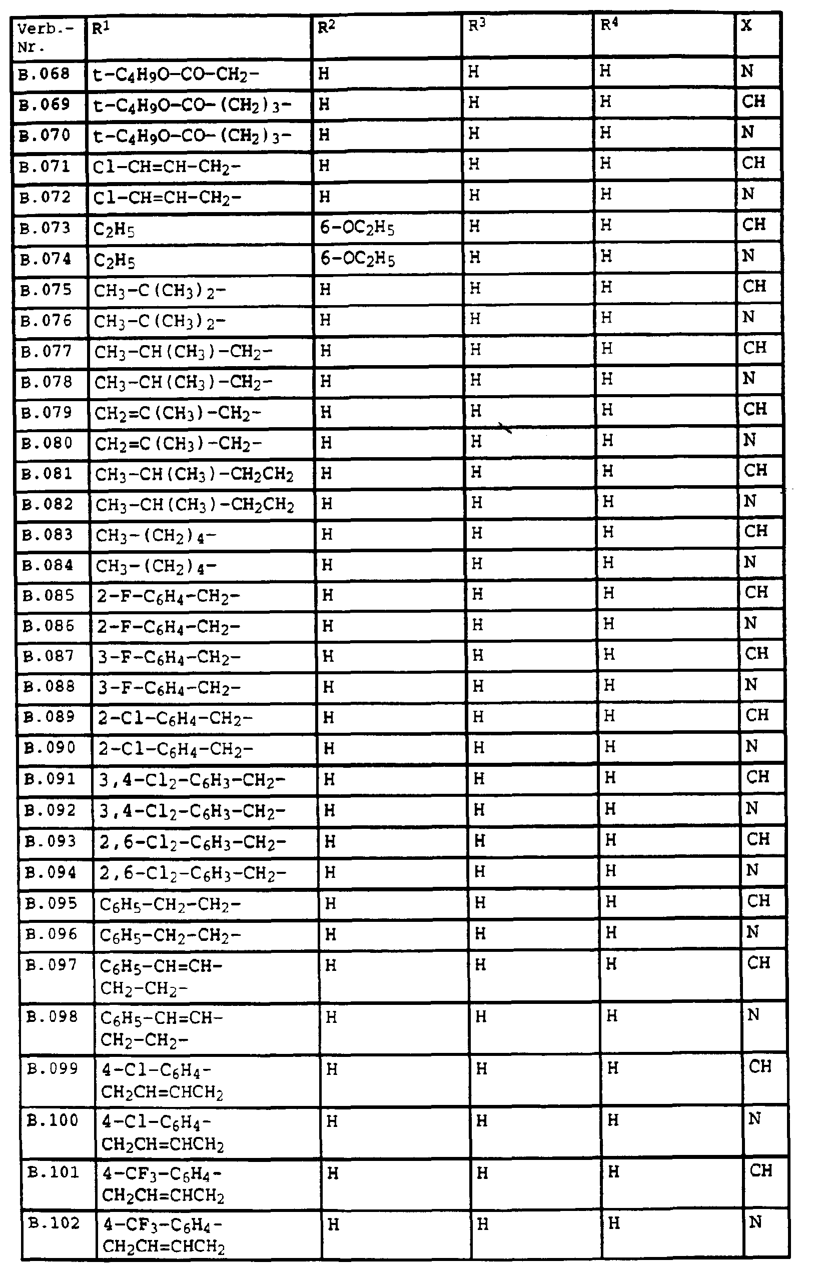

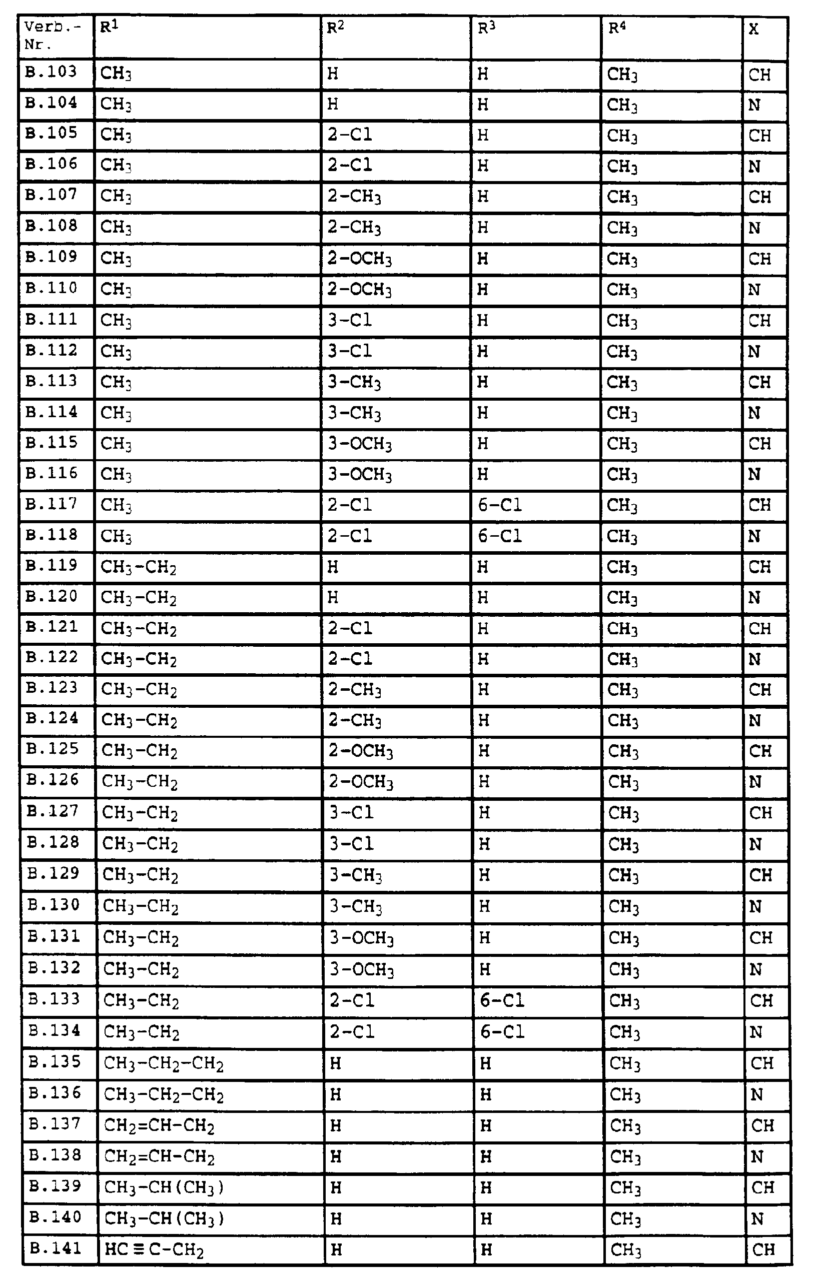

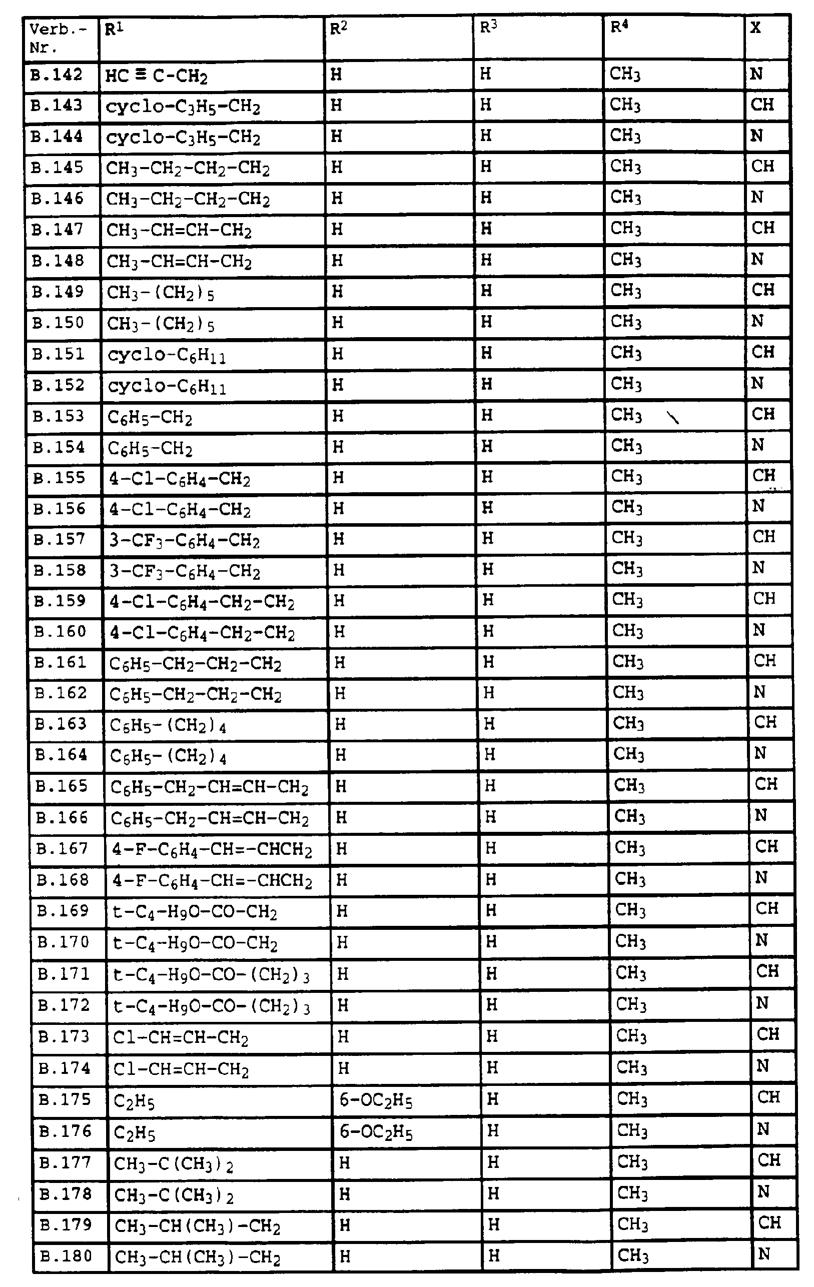

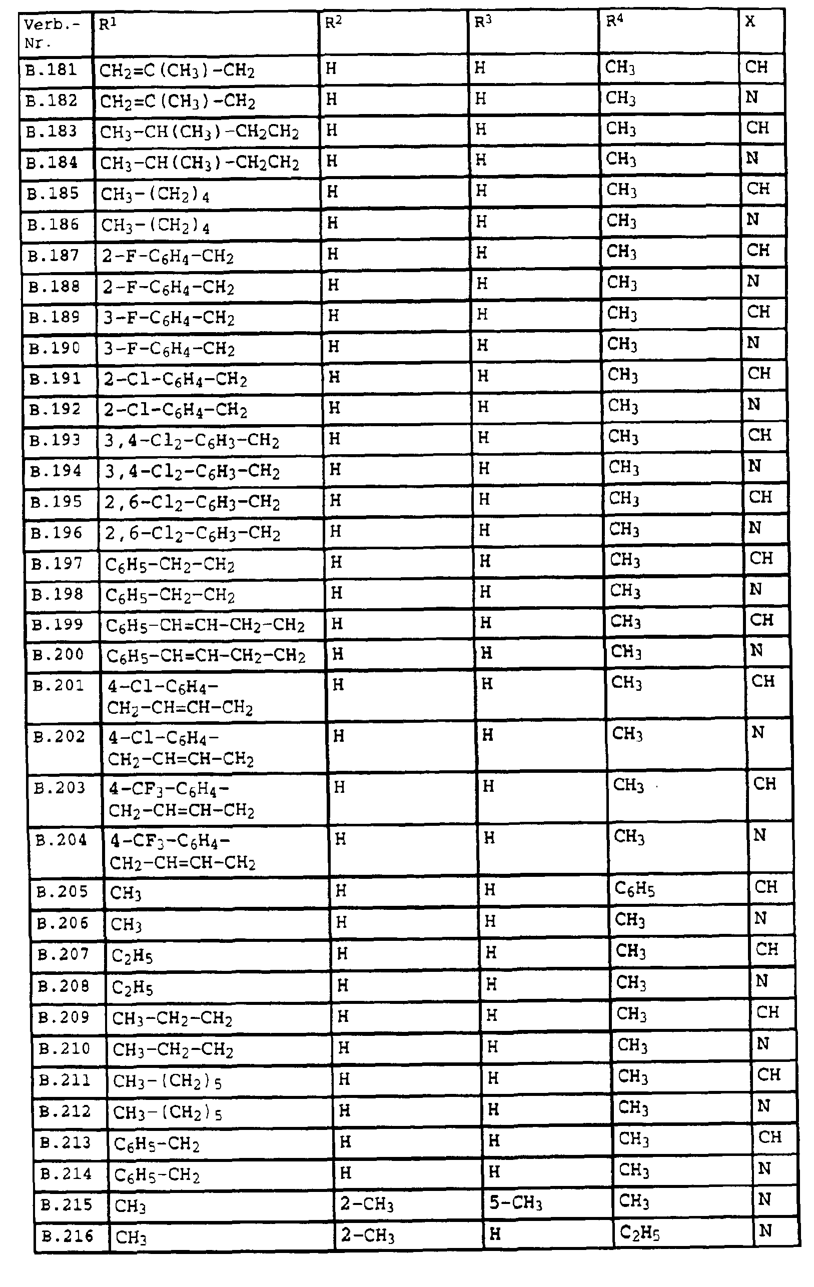

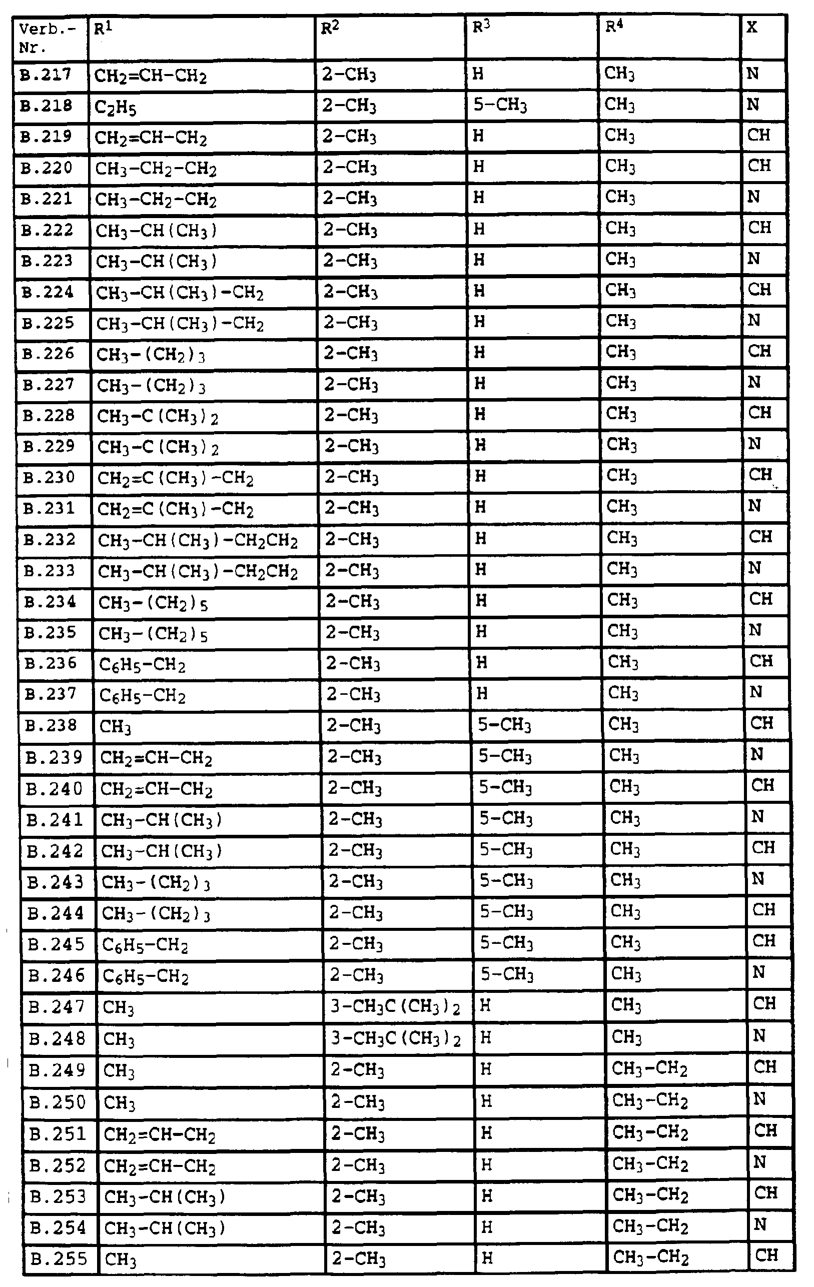

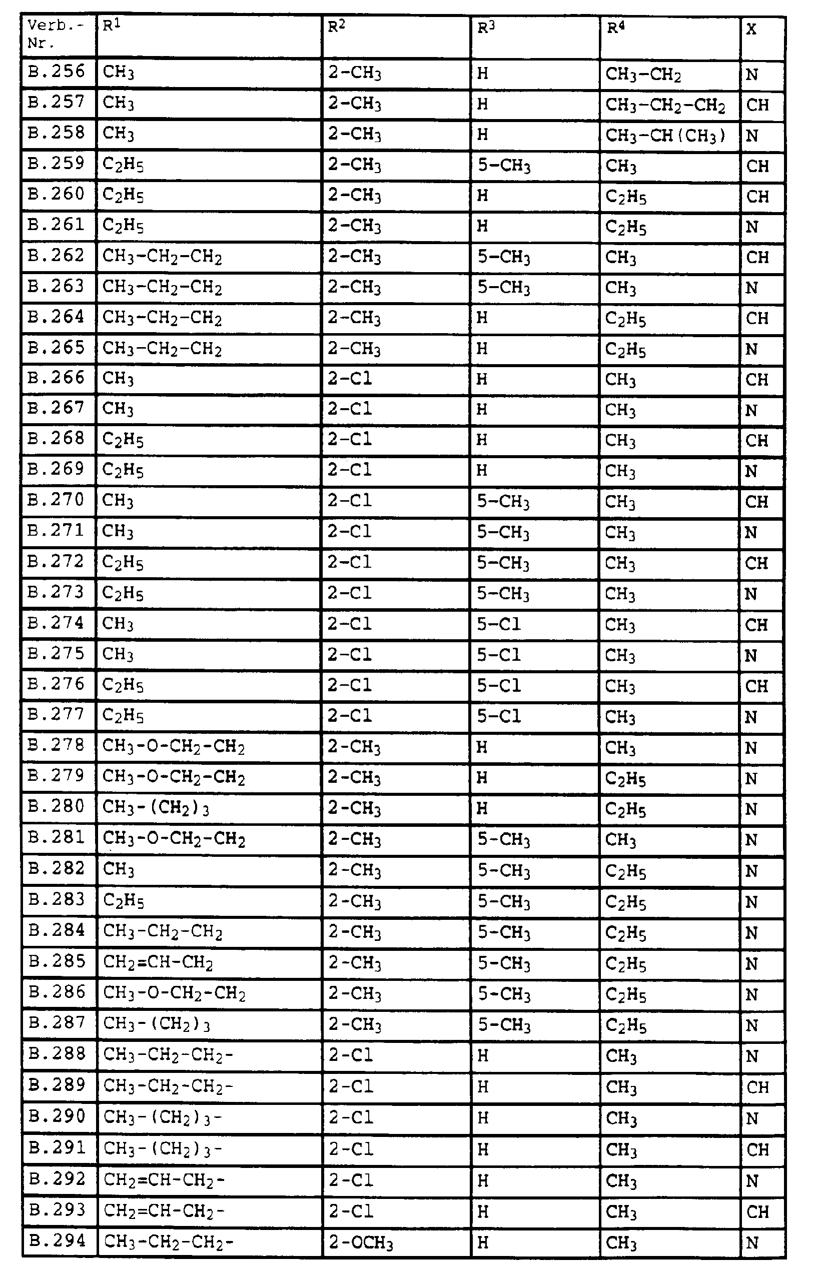

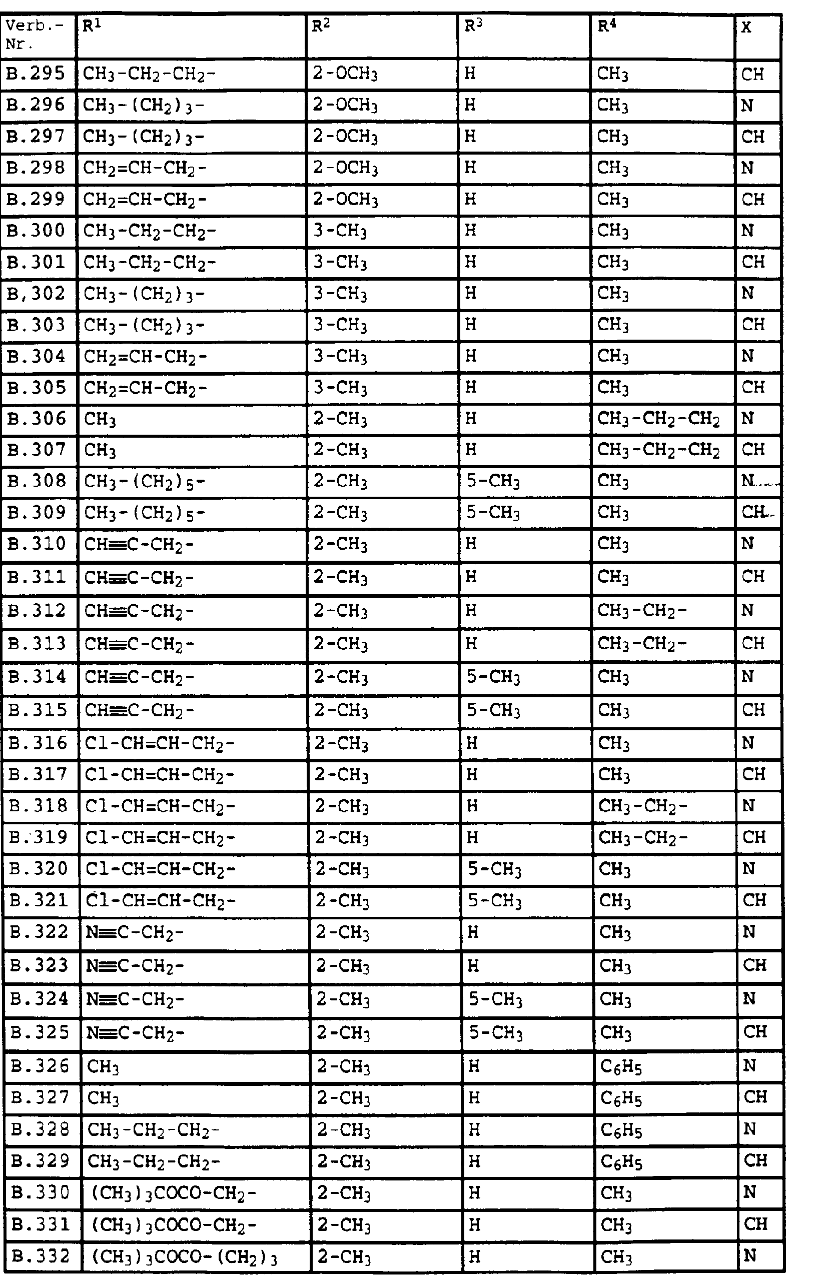

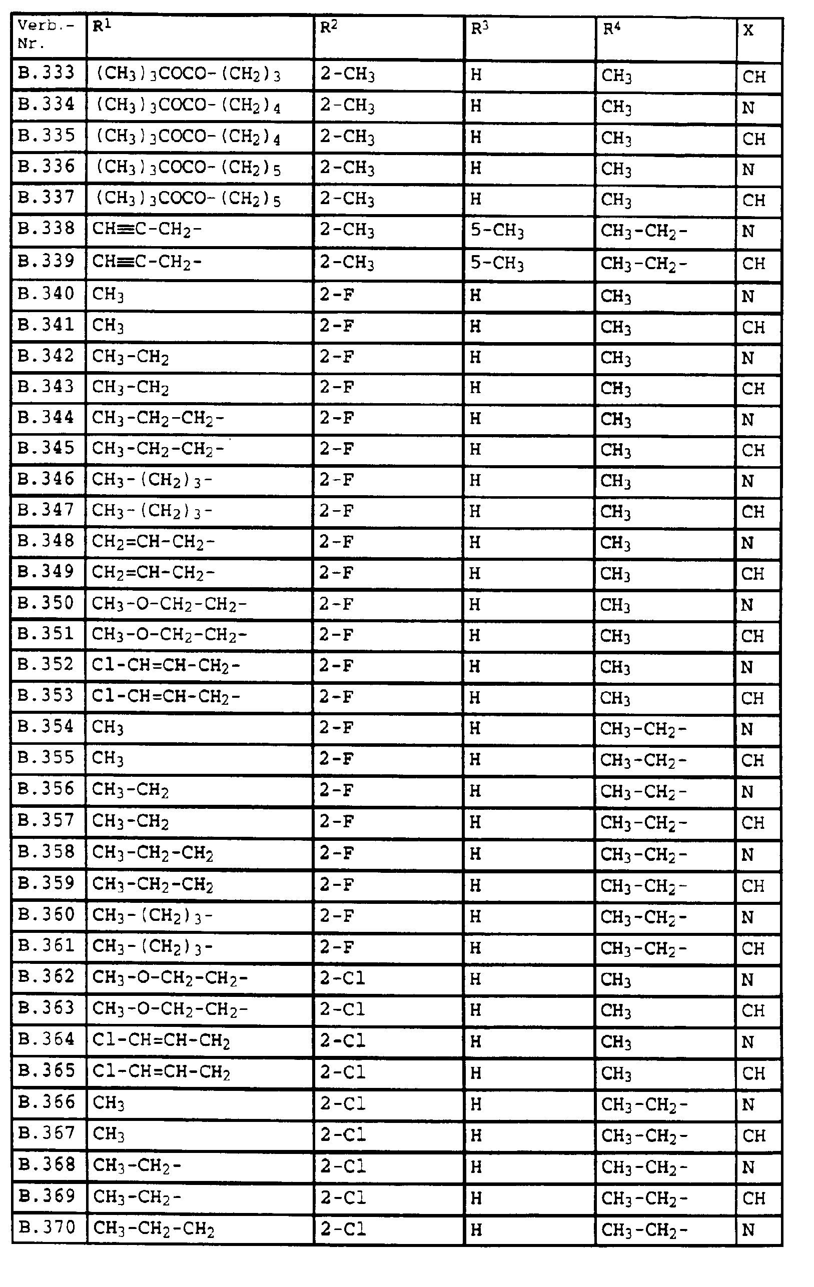

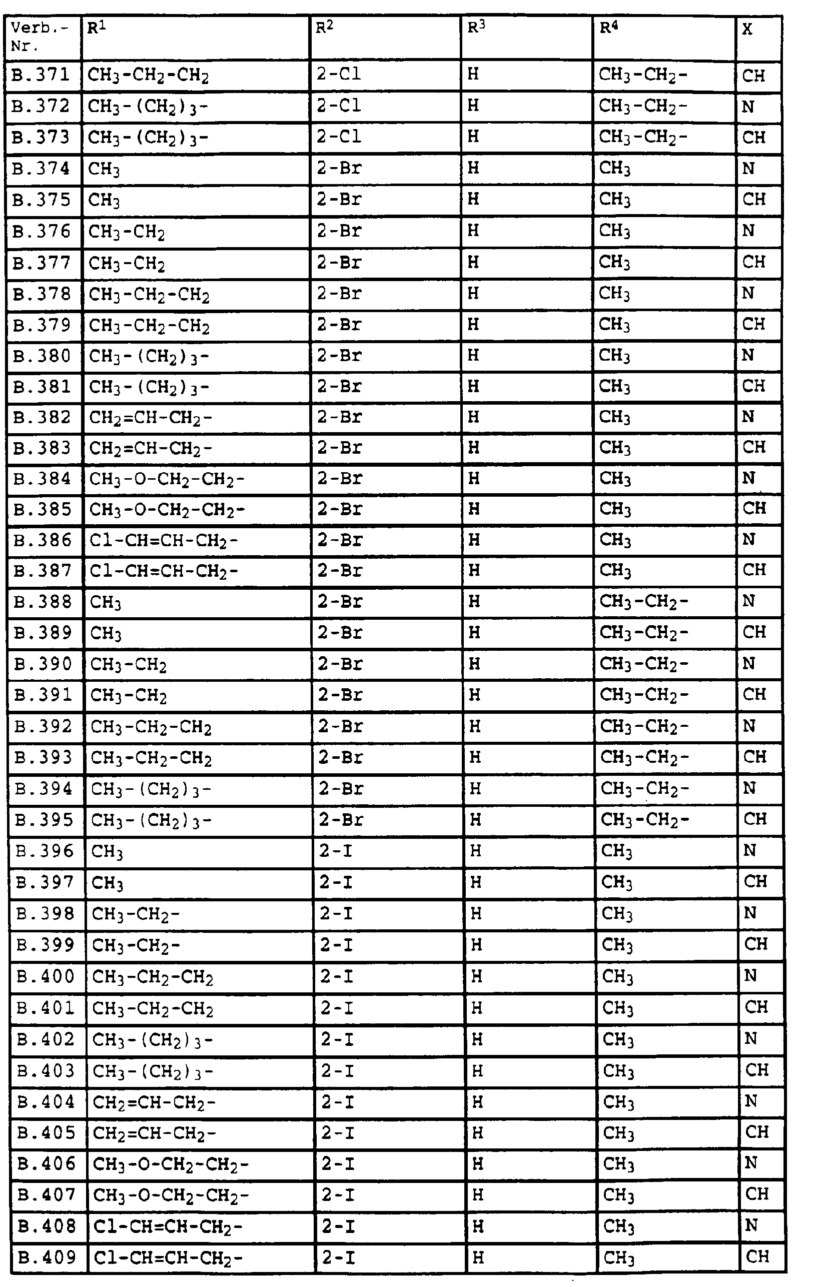

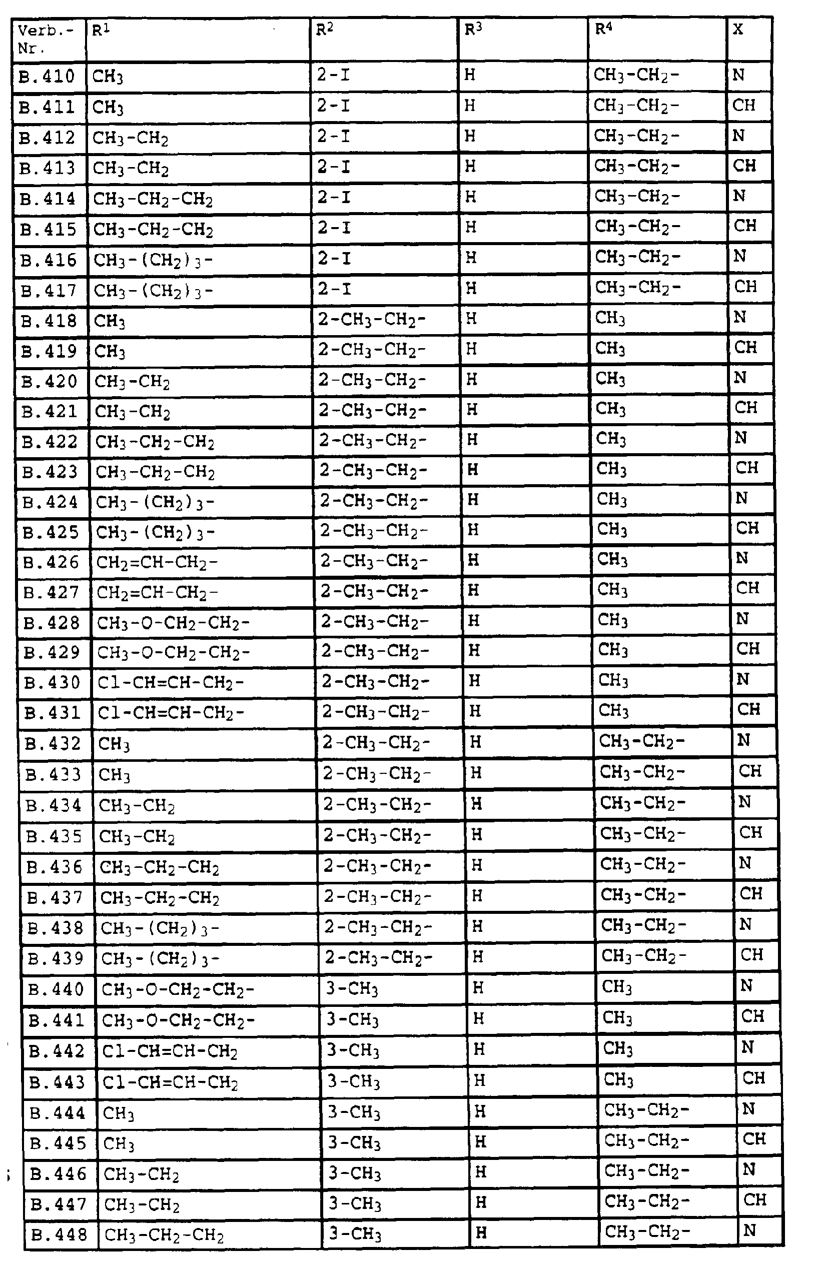

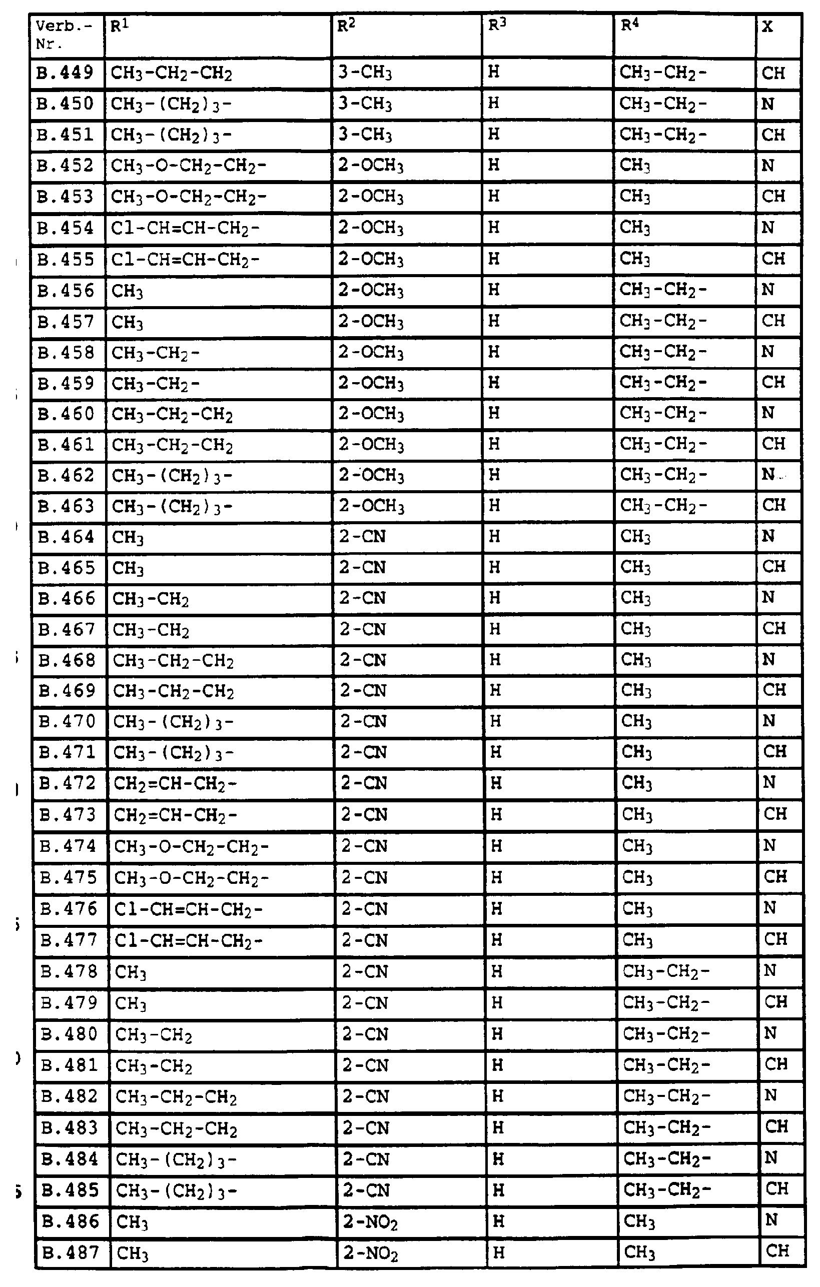

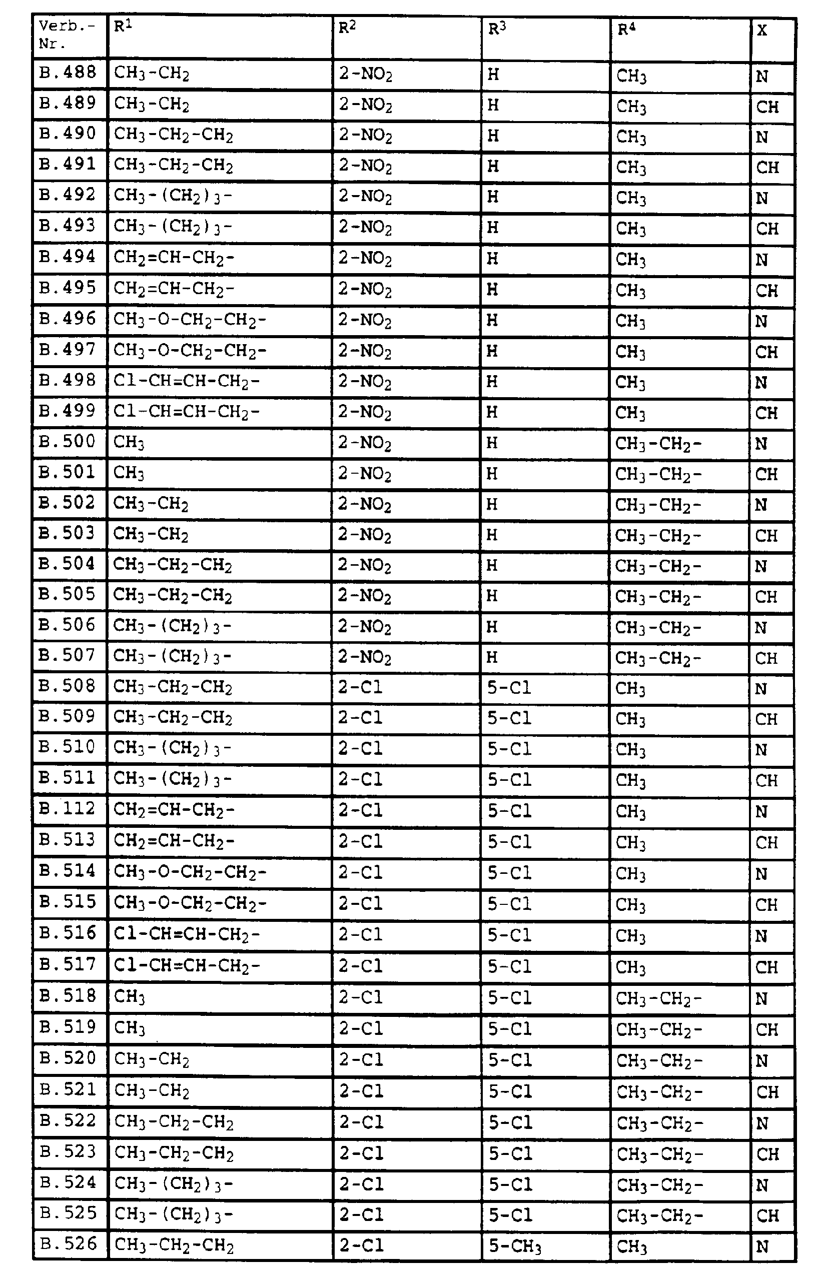

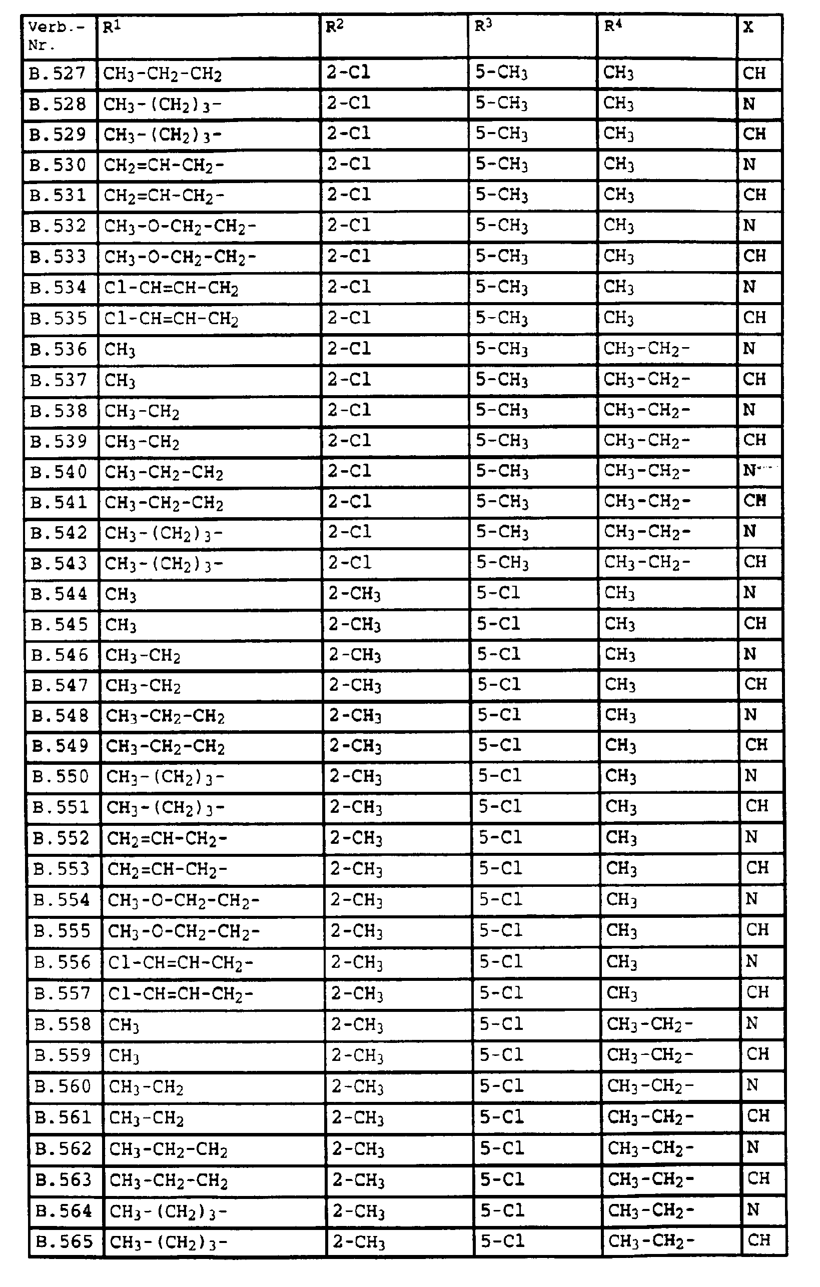

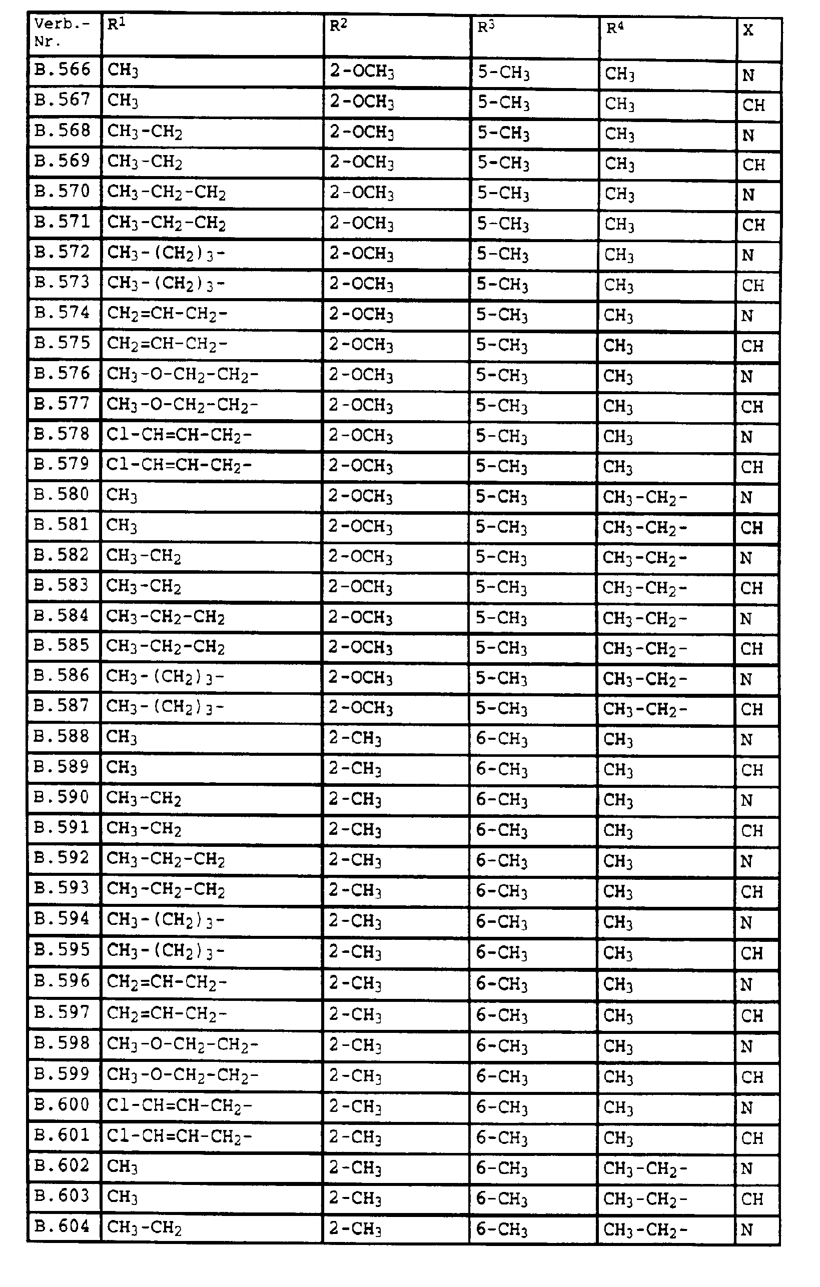

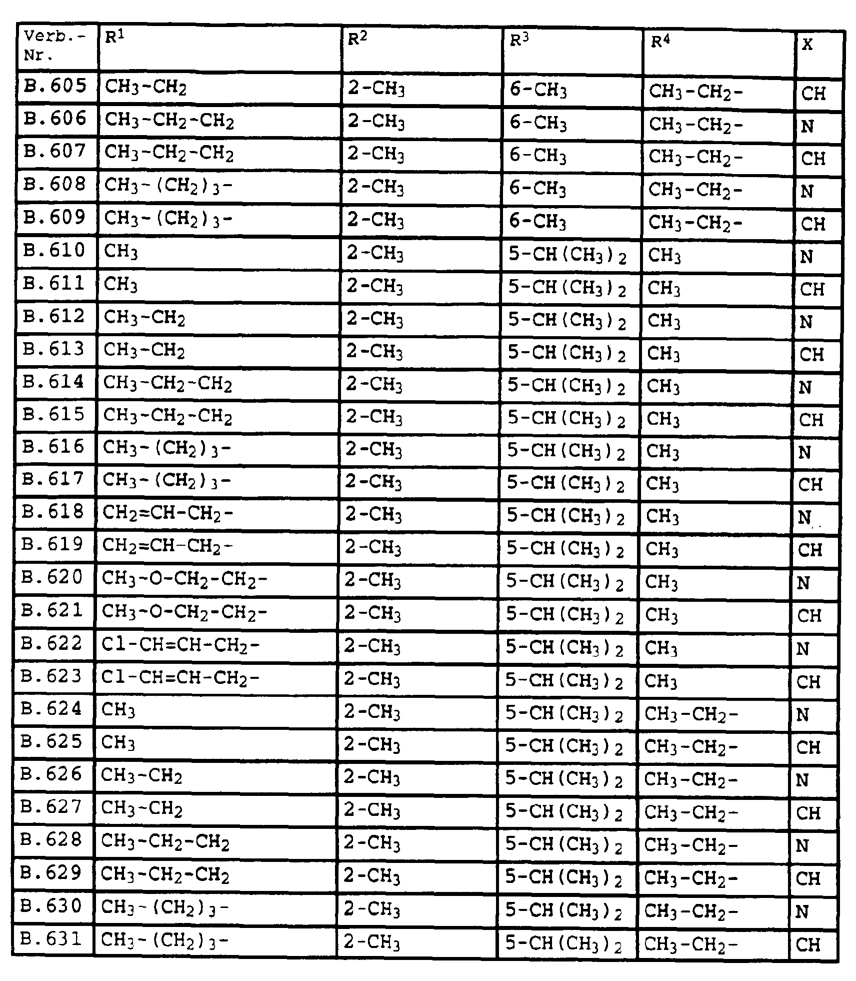

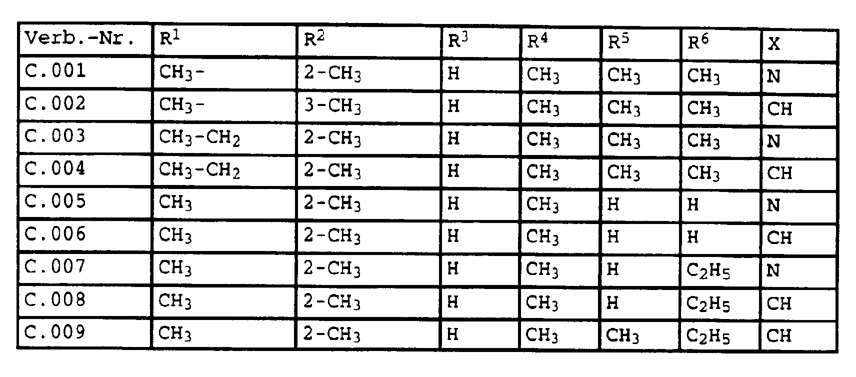

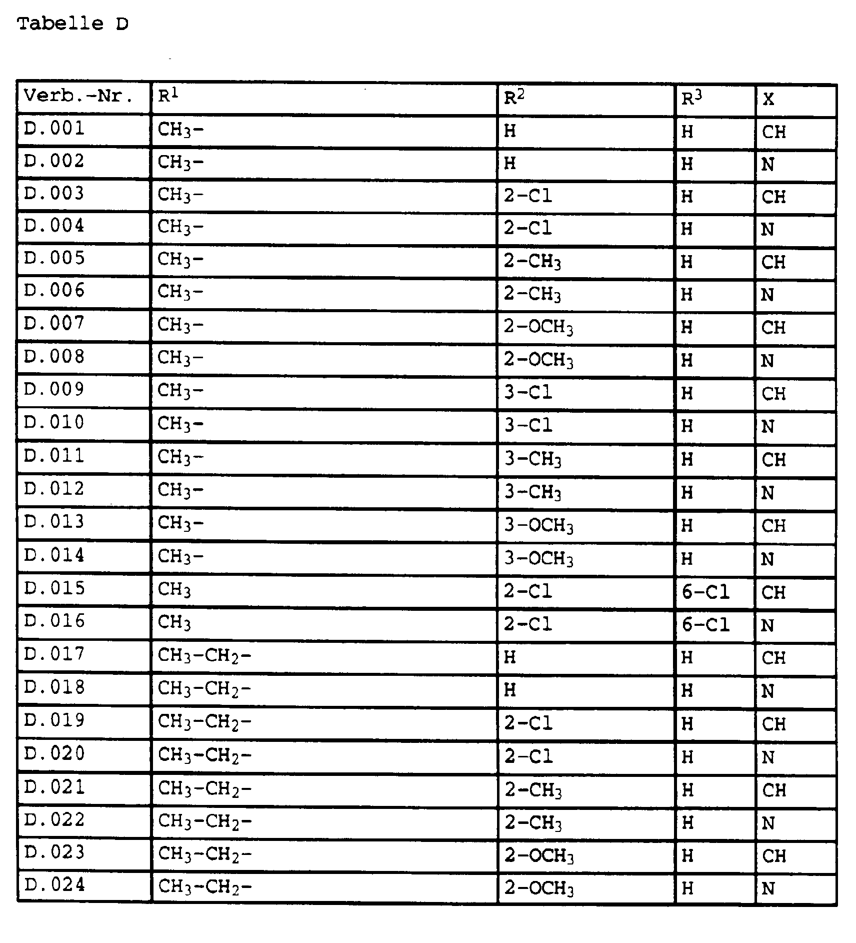

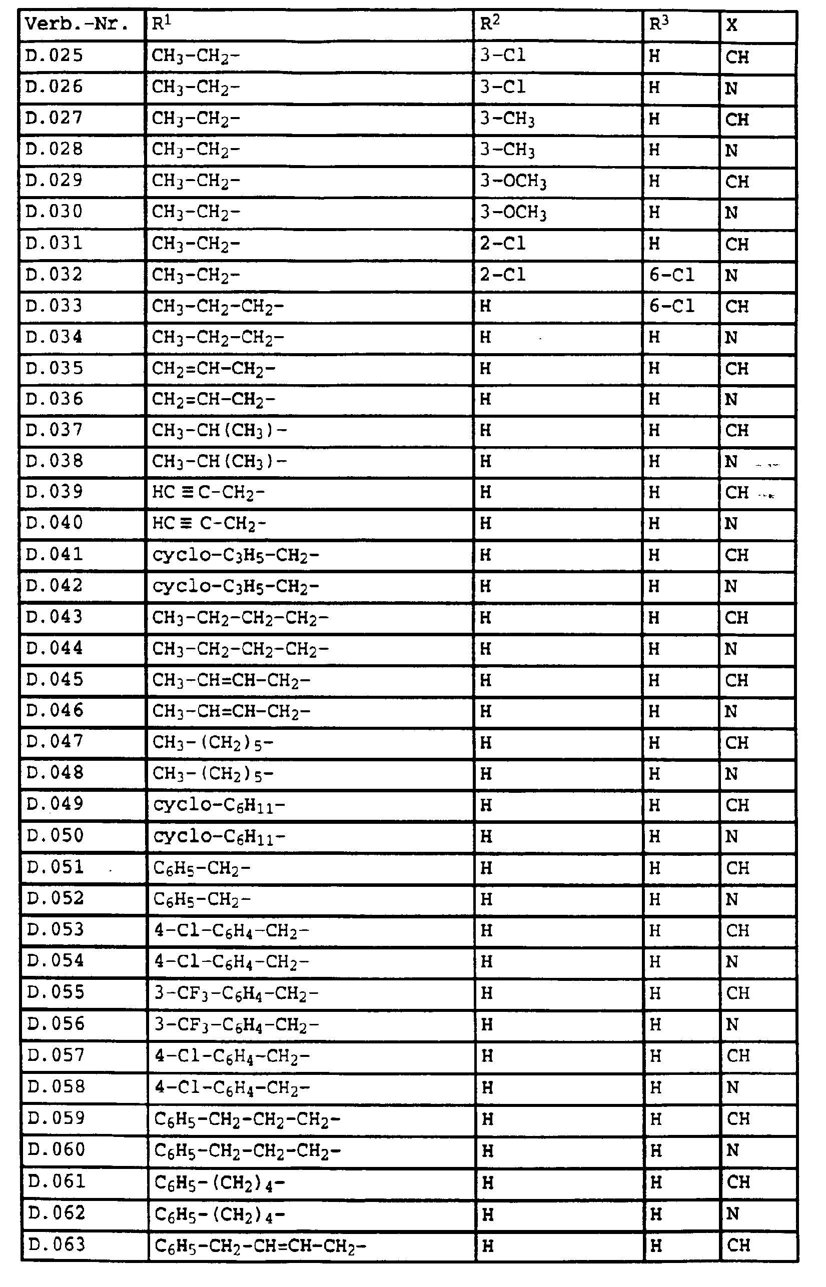

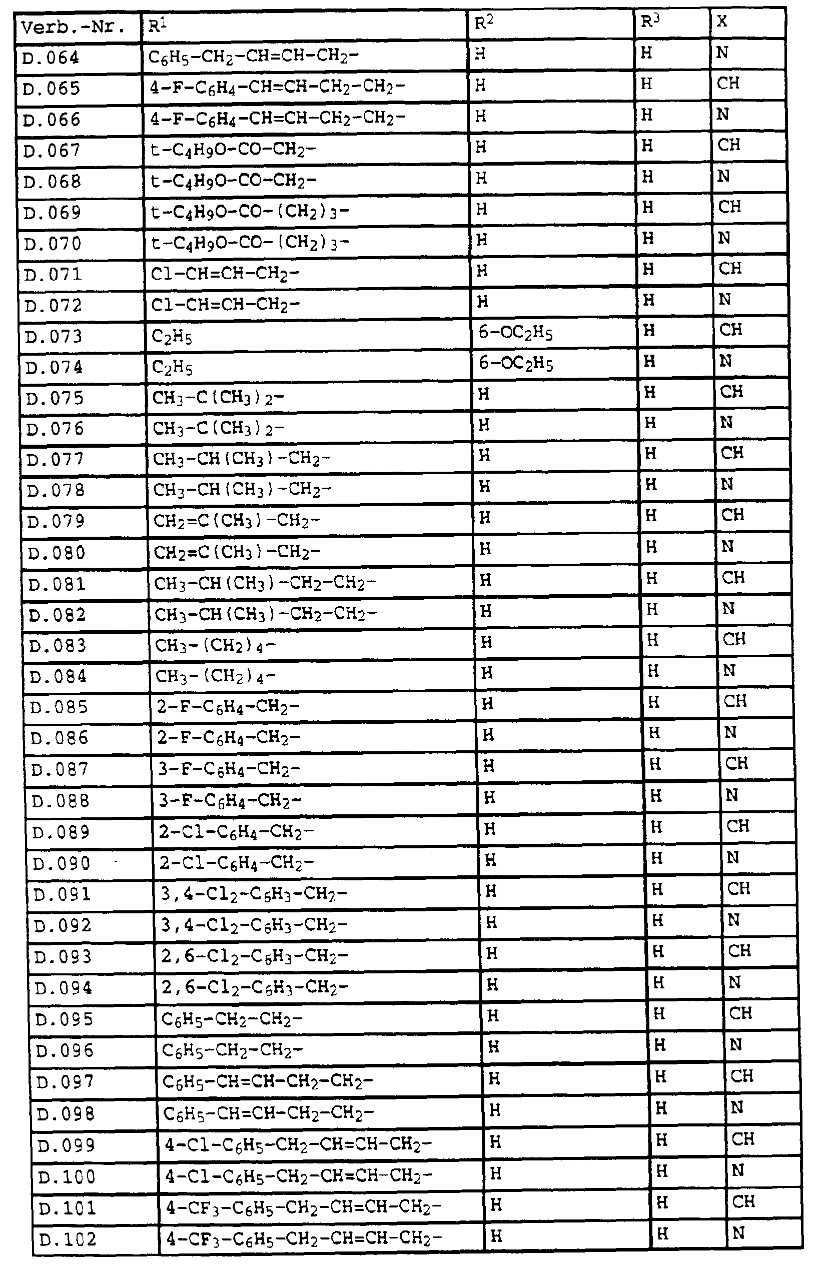

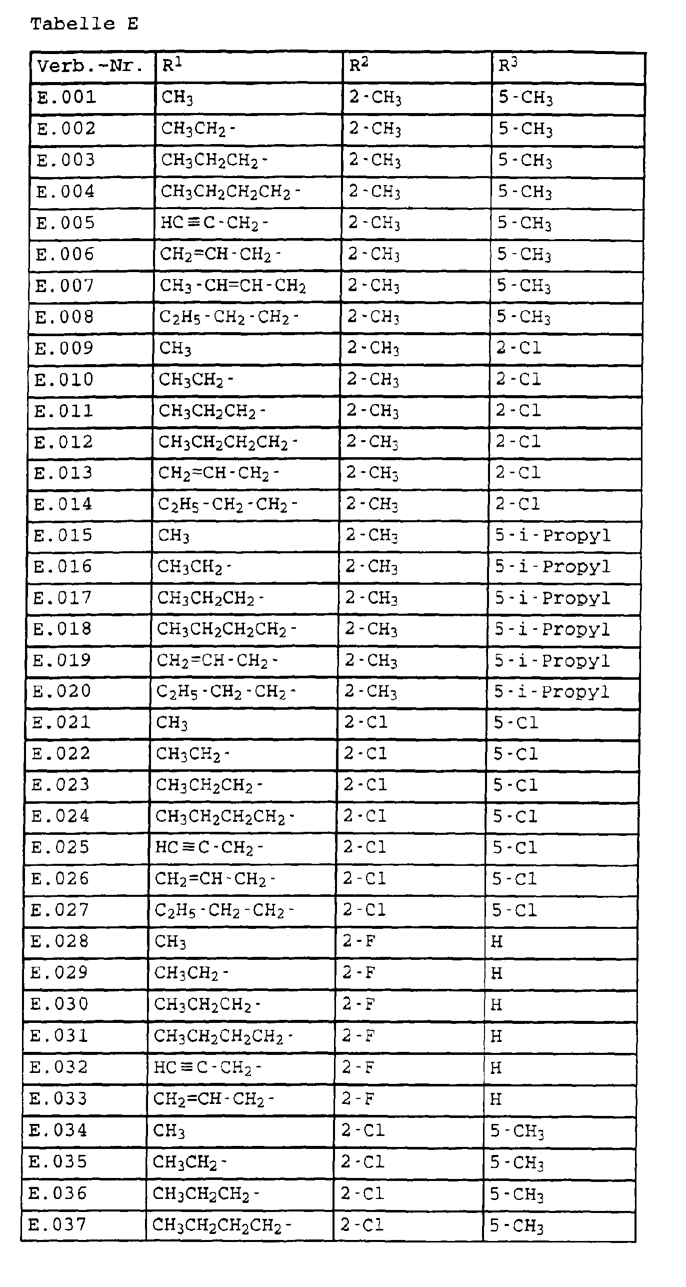

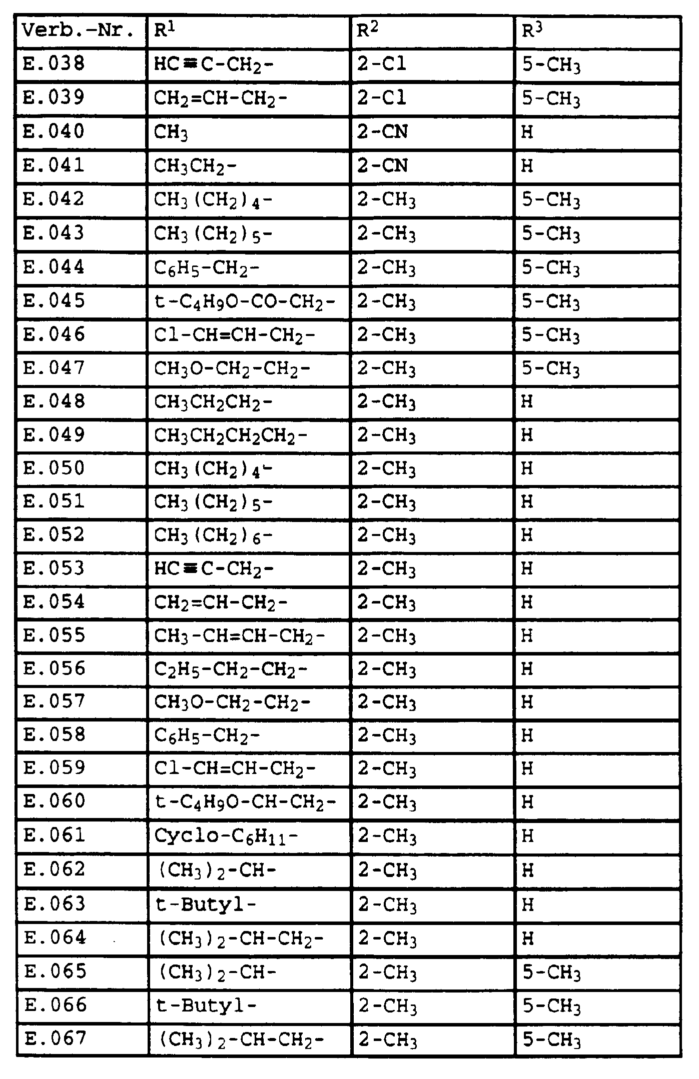

Die neuen Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus und können als Blatt- und Bodenfungizide eingesetzt werden. Sie besitzen zum Teil bemerkenswert hohe systemische Beweglichkeit und Wirksamkeit nach Boden- und insbesondere auch nach Blattapplikation.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Shaerotheca fuliginea an Kürbisgewächsen,
Podospheare leucotricha an Äpfeln,
Uncinula necator an Reben,
Puccinia-Arten an Getreide,
Rhizoctonia-Arten an Baumwolle und Rasen,
Ustilago-Arten an Getreide und Zuckkerohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen übergeführt werden, wie Lösungen, Emulsionen, Suspensionen, Stäube, Pulver, Pasten und Granulate Die Anwendungsformen richten sich nach den Verwendungszwecken; sie sollen in jedem Fall eine feine und gleichmäßige Verteilung des ortho-substituierten Benzylesters einer Cyclopropancarbonsäure gewährleisten. Die Formulierungen werden in bekannter Weise hergestellt, z. B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z B. Xylol), chlorierte Aromaten (z B. Chlorbenzole), Paraffine (z.B. Erdölfraktionen), Alkohole (z.B Methanol, Butanol), Ketone (z.B. Cyclohexanon), Amine (z.B. Ethanolamin, Dimethylformamid) und Wasser; Trägerstoffe wie natürliche Gesteinmehle (Z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z B. Polyoxyethylen-Fettalkohol-Ether, Alkylsulfonate und arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew% Wirkstoff.

Die Aufwandmengen liegen je nach Art des gewünschten Effektes zwischen 0,01 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des funziziden Wirkungsspektrums.

Die Verbindungen der Formel 1 sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta,Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Phyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthopophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hyoscyami, Phorbia antigua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Bemisia tabaci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nephotettix cincticeps, Nilaparvata lugens, Pemphigus bursarius, Perkinsiellasaccharicida, Phorodon humuli, Psylla mali, Psylla piri, Rhopalomyzus ascalonicus, Rhopalosiphum maidis, Sappahis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Retuculitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Metatetranychus(Phanonychus)ulmi, Ornithodorus moubata, Otobins megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem vis hohem Siedepunkt, wie Kerosin oder Dieselöl, fernen Kohlenteeröle sowie Öle pflanzlichen oder tierischen Ursprungs, aliphatische, cyclische und aromatische Kohlenwasserstoffe, z. B Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser in Betracht.

Wäßrige Anwendungsforman können aus Emulsionskonzentraten, Pasten der netzbaren Pulvern (Spitzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier-oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Napththalinderivaten mit Formaldehyd, Kondensationsprodukte des Naphthalins bzw der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
Granulate, z. B. Umhüllungs-, Imprägnierungs und Homogengranulate; sie können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium-und Magnesiumsulfat, Magnesiumoxid, gemahlene Kunststoffe, Düngemittel, wie z.B Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiele für die Wirkung gegen Schadpilze

Als Vergleichswirkstoffe wurden die folgenden Verbindungen benutzt 2-(2'-Methyl-phenoxymethyl)-phenylglyoxyl- sauremethylester-O-methyloxim (A) mit der Formel 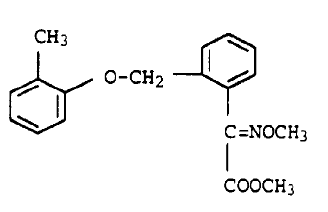 (bekannt aus EP 253 213) und 2-(2'-Methyl-4'-(melhoximinoeth-1 "-yl)-phenoxymethyl)phenylglyoxylsäuremethylester-O-methyloxim (B) mit der Formel 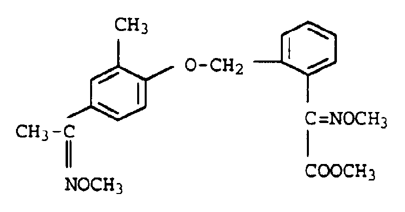 (bekannt aus EP 386 561)

### A.1 1 Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

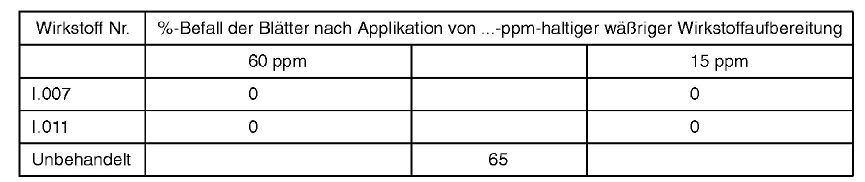

### A.2 Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt

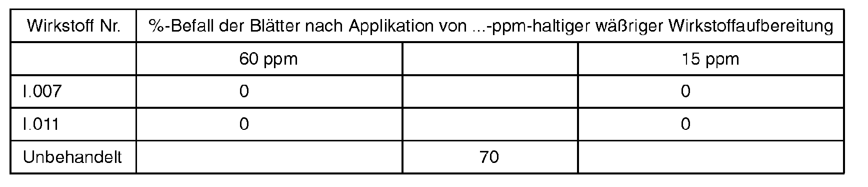

### A. Wirksamkeit gegen Bohnenrost

Blätter von Buschbohnen der Sorte "Fori" wurden mit einer wäßrigen Sporensuspension des Bohnenrostes (Uromyces appendiculatus) auf der Blattunterseite gleichmäßig besprüht. Danach wurden die Pflanzen 24 Stunden lang in einer Klimakammer mit hoher Luftfeuchtigkeit bei 19°C gehalten und anschließend im Gewächshaus bei 22 bis 25°C aufgestellt. 2 bis 3 Tage danach erfolgte die Spritzbehandlung mit den Wirkstoffen auf die untere (basale) Hälfte der Blatoberseite. Die Beurteilung des Ausmaßes der Pilzentwicklung auf den Blättern wurde 10 bis 12 Tage nach dem Besprühen vorgenommen. Durch die zeitliche und räumliche Trennung von Blattbehandlung mit den Sporen und Blattbehandlung mit den Wirkstoffen wird ein unmittelbarer Kontakt zwischen Pilz und Wirkstoff ausgeschlossen; der Fungizidwirkung muß daher die Wirkstoffaufnahme und Wirkstoffwanderung im Blatt vorausgegangen sein (systemischer Transport). Die Prüfungderverschiedenen Blattzonen erlaubt daher die Feststellung einer translaminaren oder apikalen Bewegung der getesteten Wirkstoffe im Blatt.

Das Ergebnis des Versuches zeigt, daß nach der Behandlung mit einer 50 ppm Wirkstoff enthaltenden Spritzbrühe die Wirkstoffe 1.003, 1.007 und 1.014 auf der Blattunterseite und z T auch auf dem unbehandelten Teil der Bohnenblätter eine fungizide Wirkung zeigten während die bekannten Verbindungen A und B keine fungizide Wirkung zeigten.

### Anwendungsbeispiele für die Wirkung gegen Schädlinge

Die Wirkung der Verbindungen der allgemeinen Formel gegen Schädlinge aus der Klasse der Insekten, Spinntiere und Nematoden ließ sich durch folgende Versuche zeigen:
Die Wirkstoffe wurden
   a) als 0,1 %ige Lösung in Aceton oder
   b) als 10 %ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew-% Emulphor® EL (Emulan® EL, Emulgator auf der Basis ethoxylierter Fettalkohole)
aufbereitet und entsprechend der gewünschten Konzentration mit Aceton im Fall von a) bzw mit Wasser im Fall von b) verdünnt.

Nach Abschluß der Versuche wurde die jeweils niedrigste Konzentration ermittelt, bei der die Verbindung im Vergleich zu unbehandelten Kontrollversuchen noch eine 80 - %ige Hemmung bzw Mortalität hervorriefen (Wirkschwelle bzw. Minimal-Konzentration).

### B.1 Aphis fabae (Schwarze Laus), Kontaktwirkung

Stark befallene Buschbohnen (Vicia faba) wurden mit der wäßrigen Wirkstoffaufbereitung behandelt.

Nach 24 h wurde die Mortalitätsrate bestimmt.

In diesem Test zeigten die Verbindungen 1.007, 1.011, 1.015, 1.003, 1.001, 1.017 und 1.029 Wirkschwellen von 200 bis 1000 ppm.

### B.2 Nephotettix cincticeps (Grüne Reiszikade), Kontaktwirkung

Rundfilter wurden mit der wäßrigen Wirkstoffaufbereitung behandelt und anschließend mit 5 adulten Zikaden belegt.

Nach 24 h wurde die Mortalität beurteilt.

In diesem Test zeigten die Verbindungen 1.007, l.011, l.014, 1.015, l.003_{;} 1.002, 1.004 und l.017 Wirkschwellen von 0,4 bis 0,1 mg B.3 Prodenia litura (Ägypt. Baumwollwurm), Zuchtversuch Fünf Raupen des Entwicklungsstadiums L3 (10 - 12 mm) wurden auf Standardnährboden (3,1 1 Wasser, 80 g Agar 137 g Bierhefe, 515 g Maismehl, 130 g Weizenkeime sowie übliche Zusatzstoffe und Vitamine (20 g Wessonsalz, 5 g Nipagin, 5 g Sorbin, 10 g Zellulose, 18 g Ascorbinsäure, 1 g Lutavit® blend (Vitamin), 5 ml alkoholische Biotin-Lösung)) aufgebracht, der zuvor mit der wäßrigen Wirkstoffaufbereitung benetzt worden war

Die Beobachtung erstreckte sich bis zum Schlüpfen der Falter in einem Kontrollversuch ohne Wirkstoff.

In diesem Test zeigten die Verbindungen 1.003, 1.014, 1.015 und 1.017 Wirkschwellen von 200 bis 0,1 ppm.

## Patentansprüche

1. Substituierte Oximether der allgemeinen Formel 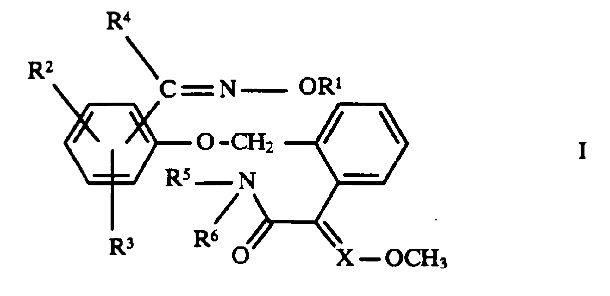 in der
R¹
C₁-C₆-Alkyl, C₃-C₆-Alkenyl, C₃-C₄-Alkinyl, C₁-C₆-Halogenalkyl, C₃-C₆-Halogenalkenyl, C₁-C₄-Alkoxy-C₁-C₆-alkyl, C₃-C₆-Cycloalkyl, C₃-C₆-Cycloalkyl-C₁-C₄-alkyl, Cyan-C₁-C₆-alkyl, C₁-C₆-Alkoxycarbonyl-C₁-C₆-alkyl, Aryl-C₁-C₆-alkyl; Heteroaryl-C₁-C₆-alkyl, Aryl-C₃-C₆-alkenyl oder Aryloxy-C₁-C₆-alkyl bedeutet, wobei der aromatische oder heteroaromatische Ring gegebenenfalls durch einen oder mehrere der folgenden Reste substituiert ist: C₁-C₄-Alkyl, C₁-C₂-halogenalkyl, C₃-C₆-Cycloalkyl, C₁-C₄-Alkoxy, C₁-C₂-Halogenalkoxy, Halogen, Aryl, Aryloxy,
R² und R3
gleich oder verschieden sind und Wasserstoff, C₁-C₄-Alkyl, C₁-C₂-Halogenalkyl, C₁-C₄-Alkoxy, C₁-C₂₋Halogenalk- oxy, Halogen, Cyano oder Nitro bedeuten,
R4
Wasserstoff, C₁-C₆-Alkyl, C₃-C₆-Cycloalkyl, C₁-C₇-Halogenalkyl oder Aryl bedeutet, wobei der aromatische Ring gegebenenfalls durch einen oder mehrere der folgenden Reste substituiert ist: C₁-C₄-Alkyl, C₁-C₂-Halogenalkyl, C₁-C₄-Alkoxy, C₁-C₂-Halogenalkoxy, Halogen, Cyano oder Nitro,
R⁵ und R6
gleich oder verschieden sind und Wasserstoff oder C₁-C₄-Alkyl bedeuten, und
X CH oder N bedeutet.

2. Verfahren zur Herstellung substituierter Oximether der allgemeinen Formel gemäß Anspruch 1 dadurch gekennzeichnet, daß man einen substituierten Oximether der allgemeinen Formel ll 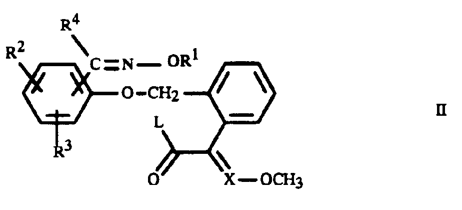 in der L für Halogen, C₁-C₄-Alkoxy oder Hydroxy steht, mit einem Amin der allgemeinen Formel lll 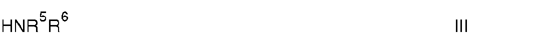 umsetzt.

3. Substituierte Oximether der allgemeinen Formel II 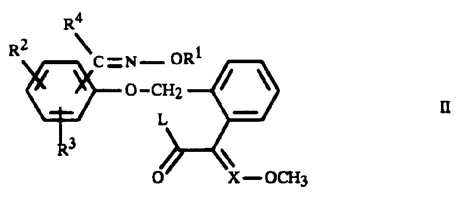 in der R^{l}, R², R³ und R⁴ die in Anspruch 1 gegebene Bedeutung haben und L für Hydroxy oder Halogen steht.

4. Fungizid, enthaltend einen inerten Trägerstoff und eine fungizid wirksame Menge eines substituierten Oximethers der allgemeinen Formel 1 gemäß Anspruch 1.

5. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines substituierten Oximethers der allgemeinen Formel l gemäß Anspruch 1 behandelt.

6. Mittel zur Bekämpfung von Schädlingen, enthaltend inerte Zusatzstoffe und eine pestizid wirksame Menge einer Verbindung der allgemeinen Formel l gemäß Anspruch I.

7. Verfahren zur Bekämpfung von Schädlingen, dadurch gekennzeichnet, daß man die Schädlinge und/oder ihren Lebensraum mit einer wirksamen Menge einer Verbindung der allgemeinen Formel l gemäß Anspruch 1 behandelt.

8. Verbindung der Formel I gemäß Anspruch 1, in der R¹ Methyl, R² (in 2-Stellung) Methyl, R⁴ und R⁶ Methyl, R³ und R⁵ Wasserstoff, X N bedeutet und der Oximinoethylrest in 4-Stellung steht.

9. Verbindung der Formel I gemäß Anspruch 1, in der R¹ Methyl, R² (in 2-Stellung) Methyl, R⁴ und R⁶ Methyl, R³ und R⁵ Wasserstoff, X CH bedeutet und der Oximinoethylrest in 4-Stellung steht.

10. Verbindung der Formel l gemäß Anspruch 1, in der R¹ Methyl, R² (in 2-Stellung) Methyl, R⁴ Cyclopropyl, R⁶ Methyl, R³ und R⁵ Wasserstoff, X N bedeutet und der Oximinorest in 4-Stellung steht.

11. Verbindung der Formel gemäß Anspruch 1, in der R¹ Methyl, R² (in 2-Stellung) Methyl, R⁴ Trifluormethyl, R⁶ Methyl, R³ und R⁵ Wasserstoff, X N bedeutet und der Oximinorest in 4-Stellung steht.

## Claims

1. A substituted oxime ether of the formula I 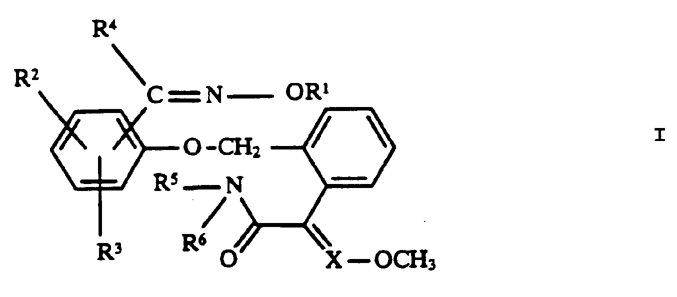 where
R¹ is C₁-C₆-alkyl, C₃-Cₛ-alkenyl, C₃-C₆-alkynyl, C₁-C₆-haloalkyl, C₃-C₆-haloalkenyl, C₁-C₆-alkoxy-C₁-C₆-alkyl, C₃-C₆-cycloalkyl, C₃-C₆-cycloalkyl-C₁-C₄-alkyl, cyano-C₁-C₆-alkyl, C₁-C₆-alkoxycarbonyl-C₁-C₆-alkyl, aryl-C₁-C₆- alkyl, helaryl-C₁-C₆-alkyl, aryl-C₃-C₆-alkenyl or aryloxy-C₁-C₆-alkyl, where the aromatic or heteroaromatic ring is unsubstituted or substituted by one or more of the following radicals: C₁-C₆-alkyl, C₁- or C₂-haloalkyl, C₃-C₆- cycloalkyl, C₁-C₄-alkoxy, C₁- or C₂-haloalkoxy, halogen, aryl or aryloxy,
R² and R³ are identical or different and are each hydrogen, C₁-C₆-alkyl, C₁- or C₂-haloalkyl, C₁-C₆-alkoxy, C₁- or C₂-haloalkoxy, halogen; cyano or nitro,
R⁴ is hydrogen, C₁-C₆-alkyl, C₃-C₆-cycloalkyl; C₁-C₇-haloalkyl or aryl, where the aromatic ring is unsubstituted or substituted by one or more of the following radicals: C₁-C₄-alkyl, C₁- or C₂-haloalkyl, C₁-C₄-alkoxy, C₁- or C₂- haloalkoxy, halogen, cyano or nitro,
R⁵ and R⁶ are identical or different and are each hydrogen or C₁-C₄-alkyl and
X is CH or N.

2. A process for the preparation of a substituted oxime ether of the formula I as claimed in claim 1, wherein a substituted oxime ether of the formula II 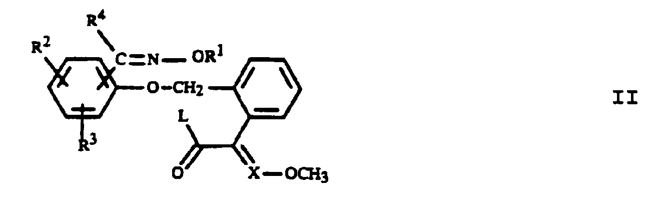 where L is halogen, C₁-C₄-alkoxy or hydroxyl, is reached with an amine of the formula lll

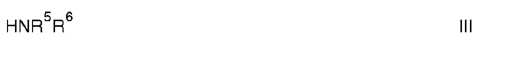

3. A substituted oxime ether of the formula II 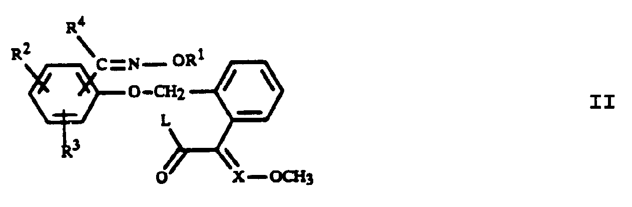 where R¹, R², R³ and R⁴ have the meanings given in claim 1 and L is hydroxyl or halogen.

4. A fungicide containing an inert carrier and a fungicidal amount of a substituted oxime ether of the formula I as claimed in claim 1.

5. A method for controlling fungi, wherein the fungi or the materials, plants or seeds threatened by fungal attack or the soil is or are treated with a fungicidal amount of a substituted oxime ether of the formula I as claimed in claim 1.

6. A pesticide containing inert additives and a pesticidal amount of a compound of the formula I as claimed in claim 1.

7. A method for controlling pests, wherein the pests and/or their habitat are or is treated with an effective amount of a compound of the formula I as claimed in claim 1.

8. A compound of the formula I as claimed in claim 1, where R¹, R² (in the 2-position), R⁴ and R⁶ are each methyl, R³ and R⁵ are each hydrogen, X is N and the oximinoethyl radical is in the 4-position.

9. A compound of the formula I as claimed in claim 1, where R¹, R² (in the 2-position), R⁴ and R⁶ are each methyl, R³ and R⁵ are each hydrogen, X is CH and the oximinoethyl radical is in the 4-position.

10. A compound of the formula I as claimed in claim 1, where R¹ is methyl, R² (in the 2-position) is methyl, R⁴ is cyclopropyl, R⁶ is methyl, R³ and R⁵ are each hydrogen, X is N and the oximino radical is in the 4-position.

11. A compound of the formula I as claimed in claim 1, where R¹ is methyl, R² (in the 2-position) is methyl, R⁴ is trifluoromethyl, R⁶ is methyl, R³ and R⁵ are each hydrogen, X is N and the oximino radical is in the 4-position.

## Revendications

1. Oximéther substitué de formule générale 1 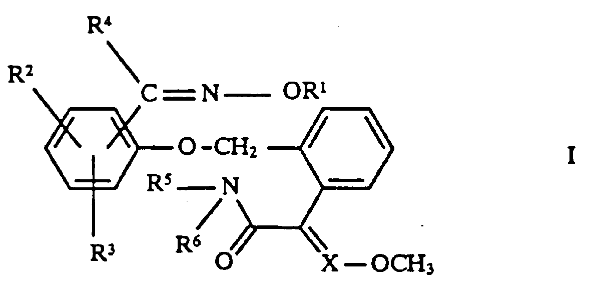 dans laquelle
R⁷ représente alkyle en C1-C6, alcényle en C3-C6, alcynyle en C3-C4, halogénalkyle en C1-C6, halogénal- cényle en C3-C6, alcoxy en C1-C4, alkyle en C1-C6, cycloalkyle en C3-C6, cycloalkyle en C3-C6, alkyle en C1-C4, cyanoalkyle en C1-C6, alcoxycarbonyle en C1-C6-alkyle en C1-C6, alkyle aryle en C1 C-6, hétéroaryle, alkyle en C1-C6, aryle alcényle en C3-C6 ou aryloxy alkyle en C1-C6, le noyau aromatique ou hétéroaroma- tique étant éventuellement substitué par un ou plusieurs des restes suivants : alkyle en C1-C4, halogénalkyle en C1-C2, cycloalkyle en C3-C6, alcoxy en C1-C4, halogénoalcoxy en C1-C2, halogène, aryle, aryloxy,
R² et R³, identiques ou différents, représentent hydrogène, alkyle en C1-C4, halogénalkyle en C1-C2, alcoxy en C1-C4, halogénalcoxy en C1-C2, halogène, cyano ou nitro,
R⁴ représente hydrogène, alkyle en C1-C6, cycloalkyle en C3-C6, halogénalkyle en C1-C7 ou aryle, le noyau aromatique étant éventuellement substitué par un ou plusieurs des restes suivants : alkyle en C1-C4, halogénalkyle en C1-C2, alcoxy en C1-C4, halogénalcoxy en C1-C2, halogène, cyano ou nitro,
R⁵ et R⁶ sont identiques ou différents et représentent hydrogène ou alkyle en C1-C4 et
X représente CH ou N

2. Procédé de préparation d'oximéther substitué de formule générale 1 selon la revendication 1, caractérisé par le fait que l'on fait réagir, un oximéther substitué de formule générale Il 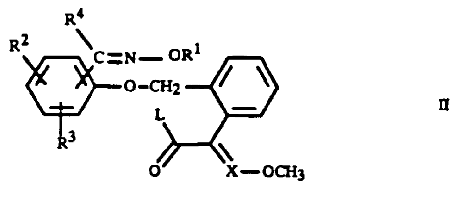 dans laquelle L est mis pour halogène, alcoxy en C1-C4 ou hydroxy, avec une amine de formule générale lll

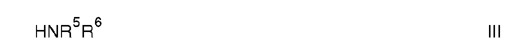

3. Oximéther substitué de formule générale Il 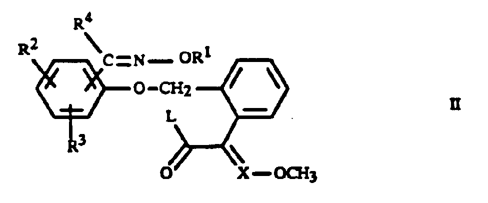 dans laquelle R¹, R², R³ et R⁴ ont la signification donnée dans la revendication 1 et L est mis pour hydroxy ou halogène.

4. Fongicide contenant un support inerte et une quantité à activité fongicide d'un oximéther substitué de formule générale l selon la revendication 1.

5. Procédé de lutte contre les champignons, caractérisé par le fait que l'on traite les champignons ou les matériaux, plantes, semences ou le sol menacés par une attaque par champignons, avec une quantité à activité fongicide d'un oximéther substitué de formule générale l selon la revendication 1.

6. Produit de lutte contre les parasites contenant un additif inerte et une quantité à activité pesticide d'un composé de formule générale I selon la revendication 1

7. Procédé de lutte contre les parasites, caractérisé par le fait que l'on traite les parasites et/ou leur biotope avec une quantité active d'un composé de formule générale l selon la revendication 1.

8. Composé de formule l selon la revendication 1, dans laquelle R¹ représente méthyle, R² méthyle (en position 2), R⁴ et R⁶ méthyle, R³ et R⁵ hydrogène, X représente N et le reste oximinoéthyle est en position 4.

9. Composé de formule l selon la revendication 1, dans laquelle R¹ représente méthyle, R² méthyle (en position 2), R⁴ et R⁶ méthyle, R³ et R⁵ hydrogène, X représente CH et le reste oximinoéthyle est en position 4.

10. Composé de formule l selon la revendication 1, dans laquelle R¹ représente méthyle, R² méthyle (en position 2), R⁴ cyclopropyle, R⁶ méthyle, R³ et R⁵ hydrogène, X représente N et le reste oximinoéthyle est en position 4.

11. Composé de formule l selon la revendication 1, dans laquelle R¹ représente méthyle, R² méthyle (en position 2), R⁴ trifluorométhyle, R⁶ méthyle, R³ et R⁵ hydrogène, X représente N et le reste oximinoéthyle est en position 4.
